# EUROPEAN PATENT SPECIFICATION

(11) **EP 4 665 445 B1**
(45) Date of publication and mention of the grant of the patent: **18.11.2026**
(21) Application number: 24703642.9
(22) Date of filing: 01.02.2024
(51) Int. Cl.: A61N 1/05, A61N 1/372, A61N 1/375, A61N 1/362

(54) **DISTAL END FIXATION FOR IMPLANTABLE MEDICAL DEVICE**
DISTALE ENDBEFESTIGUNG FÜR IMPLANTIERBARES MEDIZINISCHES GERÄT
FIXATION DE L'EXTRÉMITÉ DISTALE D'UN DISPOSITIF MÉDICAL IMPLANTABLE

(30) Priority: 15.02.2023 US 202363485174 P; 26.01.2024 US 202463625461 P
(43) Date of publication of application: 24.12.2025
(73) Proprietor: Medtronic, Inc., Minneapolis, Minnesota 55432 (US)
(72) Inventor: HAYDEN, Kaileigh E., Minneapolis, Minnesota 55432 (US); ANDERSON, Thomas A., Minneapolis, Minnesota 55432 (US); SACK, Kevin L., Minneapolis, Minnesota 55432 (US); O'BRIEN, Kevin T., Minneapolis, Minnesota 55432 (US); HILPISCH, Kathryn E., Minneapolis, Minnesota 55432 (US); HAMACK, Jason D., Minneapolis, Minnesota 55432 (US)
(74) Representative: Zimmermann & Partner Patentanwälte mbB
(86) International application number: PCT/IB2024/050922
(87) International publication number: WO 2024/170982

(56) References cited:
- WO-A1-2023/283060
- US-A1- 2021 154 477
- US-A1- 2021 275 824
- US-B2- 11 541 232
- US-B2- 8 903 513

## Description

### TECHNICAL FIELD

The disclosure relates to medical devices, and more particularly to configuration of electrodes of medical devices.

### BACKGROUND

Various types of implantable medical devices (IMDs) have been implanted for treating or monitoring one or more conditions of a patient. Such IMDs may be adapted to monitor or treat conditions or functions relating to heart, muscle, nerve, brain, stomach, endocrine organs or other organs and their related functions. Such IMDs may be associated with leads that position electrodes at a desired location, or may be leadless with electrodes integrated with and/or attached to the device housing. These IMDs may have the ability to wirelessly transmit data either to another device implanted in the patient or to another instrument located externally of the patient, or both.

A cardiac pacemaker is an IMD configured to deliver cardiac pacing therapy to restore a more normal heart rhythm. Such IMDs sense the electrical activity of the heart, and deliver cardiac pacing based on the sensed electrical activity, via electrodes. Some cardiac pacemakers are implanted a distance from the heart and coupled to one or more leads that intravascularly extend into the heart to position electrodes with respect to cardiac tissue. Some cardiac pacemakers are sized to be completely implanted within one of the chambers of the heart and may include electrodes integrated with or attached to the device housing rather than leads. Some cardiac pacemakers provide dual chamber functionality, by sensing and/or stimulating the activity of both atria and ventricles, or other multi-chamber functionality. A cardiac pacemaker may provide multi-chamber functionality via leads that extend to respective heart chambers, or multiple cardiac pacemakers may provide multi-chamber functionality by being implanted in respective chambers.
US 11,541,232 B2 relates to an electrode configuration for a medical device.

### SUMMARY

Claim 1 is drawn to a device. The methods described herein are not claimed as such but are useful for understanding the claimed subject-matter.

In general, this disclosure is directed to implantable medical devices (IMDs) configured to sense and deliver electrical signals to tissue of a patient via a plurality of electrodes. More particularly, this disclosure is directed to IMDs having one or more ramps extending from a distal end of an elongated housing of the IMD. An electrode may be disposed on and/or in a distal surface of the one or more ramps. The ramps may provide a separation distance between the electrode and the distal end of the elongated housing, e.g., to improve electrical contact between the electrode and tissue of the patient, to improve functionality of the electrode. The ramps may also provide an impediment to unintended rotation and/or dislodgement of the IMD from the tissue of the patient.

In some examples, a single IMD is implanted in one chamber of a heart of the patient and is able to sense in and/or deliver cardiac pacing to more than one chamber, which may avoid the need for a leaded device or multiple smaller devices to provide such functionality, which may reduce the amount of material implanted within the patient. In some examples, such an implantable medical device includes a distal electrode that is configured to penetrate through wall tissue of the heart chamber in which the device is implanted, and into wall tissue of another heart chamber. In addition to the distal electrode, the device includes a reference electrode and one or more proximal electrodes configured to contact the wall tissue of the heart chamber. The distal electrode may be a helix configured to penetrate tissue of the patient. The distal electrode may be configured to sense in and/or deliver cardiac pacing to one chamber of the heart and the one or more proximal electrodes may be configured to sense in and/or deliver cardiac pacing to another separate chamber of the heart.

The IMD may include an elongated housing extending from a proximal end to a distal end. The electrodes can be connected to a distal end of the elongated housing. As described herein, the IMD may include one or more ramps disposed on the distal end of the elongated housing and extending distally from the distal end. A proximal electrode may be disposed on one of the one or more ramps of the IMD. Placement of the proximal electrode on the ramp may improve performance of the proximal electrode e.g., by improving contact between the proximal electrode and the tissue relative to another electrode placed on the distal end of the elongated housing. The improved contact may improve performance by, e.g., reducing pacing thresholds for the proximal electrode. The ramp may further improve performance of the IMD by preventing unintended rotation and/or dislodgment of the IMD from tissue of the patient, thereby improving the consistency of the sensing and/or pacing capabilities of the electrodes.

In some examples, this disclosure is directed to a device comprising: an elongated housing extending from a proximal end to a distal end, the elongated housing being configured to be implanted wholly within a chamber of a heart; a first electrode extending distally from the distal end of the elongated housing, the first electrode comprising: an elongated body defining a helix; a ramp extending distally from the distal end of the elongated housing; and a second electrode disposed on the ramp, wherein the ramp is configured to promote contact between the second electrode and wall tissue of the chamber without penetration of the wall tissue of the chamber by the second electrode, and wherein the ramp is configured to separate the second electrode from the distal end of the elongated housing by a fixed distance.

In some examples, this disclosure is directed to a fixation device comprising: an elongated body extending distally from a distal end of an implantable medical device, the elongated body comprising: a proximal end located at the distal end of the implantable medical device; and a helix extending distally from the proximal end and defining one or more coils, wherein a distal end of the helix is configured to penetrate into tissue of a patient; and a ramp extending distally from the distal end of the implantable medical device, wherein the ramp defines a partial helix and a distal surface, wherein the ramp is configured to contact the tissue without penetrating the tissue, and wherein the ramp is configured to separate the distal surface from the distal end of the elongated housing by a fixed distance.

In some examples, this disclosure is directed to a method comprising: delivering cardiac pacing from a device to a heart, wherein the device comprises: an elongated housing extending from a proximal end to a distal end, the elongated housing being configured to be implanted wholly within a first chamber of a heart; a first electrode extending distally from the distal end of the elongated housing, the first electrode comprising an elongated body defining a helix; a ramp extending distally from the distal end of the elongated housing; a second electrode disposed on the ramp, wherein the ramp is configured to promote contact between the second electrode and wall tissue of the first chamber without penetration of the wall tissue of the first chamber by the second electrode, and wherein the ramp is configured to separate the second electrode from the distal end of the elongated housing by a fixed distance; and signal generation circuitry within the elongated housing, the signal generation circuitry being coupled to the first electrode and the second electrode, wherein delivering the cardiac pacing comprises: delivering cardiac pacing to the second chamber of the heart via the first electrode; and delivering cardiac pacing to the first chamber of the heart via the second electrode.

In some examples, this disclosure is directed to A device comprising: an elongated housing extending along a central longitudinal axis from a proximal end to a distal end, the elongated housing being configured to be implanted wholly within a chamber of a heart, the elongated housing having a distal end major surface; a fixation system comprising a helix extending distally beyond the distal end major surface by a first distance, and a first prominence extending distally beyond the distal end major surface by a second distance, the second distance being shorter than the first distance; wherein: the helix defines a first helix location at which the helix exits the distal end major surface; a first axis is defined by the first helix location and a center of the housing distal end; a second axis is defined by the first prominence and the center of the housing distal end; and the first axis and the second axis are separated by an angle, wherein a second helix location at the angle is separated from the distal end major surface by a threshold distance.

In some examples, this disclosure is directed to a method comprising implanting a device described herein in a first chamber of a heart of a patient, enabling the first electrode to at least one of sense electrical activity of, and deliver pacing pulses to, a second chamber of the heart of the patient which is different from the first chamber, and enabling the second electrode to at least one of sense electrical activity of, and deliver pacing pulses to, the first chamber.

In some examples, this disclosure is directed to a device comprising: an elongated housing extending from a proximal end to a distal end, the elongated housing being configured to be implanted wholly within a chamber of a heart; a first electrode extending distally from the distal end of the elongated housing, the first electrode comprising an elongated body defining a helix configured to penetrate wall tissue of the chamber; a second electrode disposed on the distal end of the elongated housing, the second electrode being configured to contact the wall tissue of the chamber without penetrating the wall tissue; and a ramp extending distally from the distal end of the elongated housing, wherein the ramp is configured to inhibit unintended rotation of the helix within the wall tissue of the chamber.

In some examples, this disclosure describes a device comprising: an elongated housing extending from a proximal end to a distal end, the elongated housing being configured to be implanted wholly within a chamber of a heart; a first electrode extending distally from the distal end of the elongated housing, the first electrode comprising: an elongated body defining a helix; a ramp extending distally from the distal end of the elongated housing, wherein the ramp is configured to, without penetration of wall tissue of the chamber, inhibit unintended rotation of the elongated body within the wall tissue; and a second electrode disposed on the distal end of the housing, wherein the second electrode is configured to contact the wall tissue of the chamber without penetrating the wall tissue when the device is implanted within the chamber.

In some examples, this disclosure describes a method comprising: delivering cardiac pacing from a device to a heart, wherein the device comprises: an elongated housing extending from a proximal end to a distal end, the elongated housing being configured to be implanted wholly within a first chamber of a heart; a first electrode extending distally from the distal end of the elongated housing, the first electrode comprising an elongated body defining a helix; a ramp extending distally from the distal end of the elongated housing; a second electrode disposed on the distal end of the housing, wherein the second electrode is configured to contact the wall tissue of the chamber without penetrating the wall tissue when the device is implanted within the chamber; and signal generation circuitry within the elongated housing, the signal generation circuitry being coupled to the first electrode and the second electrode, wherein delivering the cardiac pacing comprises: delivering cardiac pacing to the second chamber of the heart via the first electrode; and delivering cardiac pacing to the first chamber of the heart via the second electrode.

In some examples, this disclosure describes a device comprising: an elongated housing extending from a proximal end to a distal end, the elongated housing being configured to be implanted wholly within a chamber of a heart; a first electrode extending distally from the distal end of the elongated housing, the first electrode comprising: an elongated body defining a helix; a ramp extending distally from the distal end of the elongated housing, wherein the ramp is configured to, without penetration of wall tissue of the chamber, inhibit unintended rotation of the elongated body within the wall tissue; and a second electrode disposed on the distal end of the housing, wherein at least a portion of the second electrode is disposed on a distalmost surface of the ramp, wherein the second electrode is configured to contact the wall tissue of the chamber without penetrating the wall tissue when the device is implanted within the chamber.

This summary is intended to provide an overview of the subject matter described in this disclosure. It is not intended to provide an exclusive or exhaustive explanation of the methods and systems described in detail within the accompanying drawings and description below.

### BRIEF DESCRIPTION OF DRAWINGS

The details of one or more examples of this disclosure are set forth in the accompanying drawings and the description below. Other features, objects, and advantages of this disclosure will be apparent from the description and drawings, and from the claims.
FIG. 1 is a conceptual diagram illustrating an example device implanted in the heart of a patient, in accordance with one or more aspects of this disclosure.
FIG. 2A is a perspective diagram illustrating the example device of FIG. 1 with a ramp and an electrode on a distal surface of the ramp.
FIG. 2B is a perspective diagram illustrating a top-down view of the example device of FIG. 2A.
FIG. 2C is a perspective diagram illustrating a side view of the distal end of the example device of FIG. 2A.
FIG. 3 is a functional block diagram illustrating an example configuration of the IMD of FIGS. 1-2C, in accordance with one or more aspects of this disclosure.
FIG. 4 is a conceptual diagram of the device of FIGS. 1-3 implanted at a target implant site.
FIG. 5 is a perspective diagram illustrating another example distal end of example device of FIG. 1.
FIG. 6A is a perspective diagram illustrating an example distal end of example device of FIG. 1 with two ramps.
FIG. 6B is a perspective diagram illustrating an example distal end of example device of FIG. 5 with two ramps.
FIG. 7 is a flow diagram illustrating an example process for sensing a cardiac electrical signal and delivering cardiac pacing therapy to a heart of a patient via an example device of any of FIGS. 1-6.
FIG. 8A is a perspective diagram illustrating another example distal end of the example device of FIG. 1.
FIG. 8B is a perspective diagram illustrating a top-down view of the example distal end of the example device of FIG. 8A.
FIG. 8C is a perspective diagram illustrating a side view of the distal end of the example device of FIG. 8A.
FIG. 9A is a perspective diagram illustrating another example distal end of the example device of FIG. 1.
FIG. 9B is a perspective diagram illustrating a side view of the distal end of the example device of FIG. 9A.

### DETAILED DESCRIPTION

In general, this disclosure is directed to configurations of electrodes of implantable medical devices (IMDs). More particularly, this disclosure is directed to IMDs having a plurality of electrodes configured to sense electrical signals from and to deliver electrical stimulation (e.g., cardiac pacing) to tissue of a patient. A physical arrangement of plurality of electrodes on the IMD may define a plurality of reference axes defining a three-dimensional (3D) coordinate system.

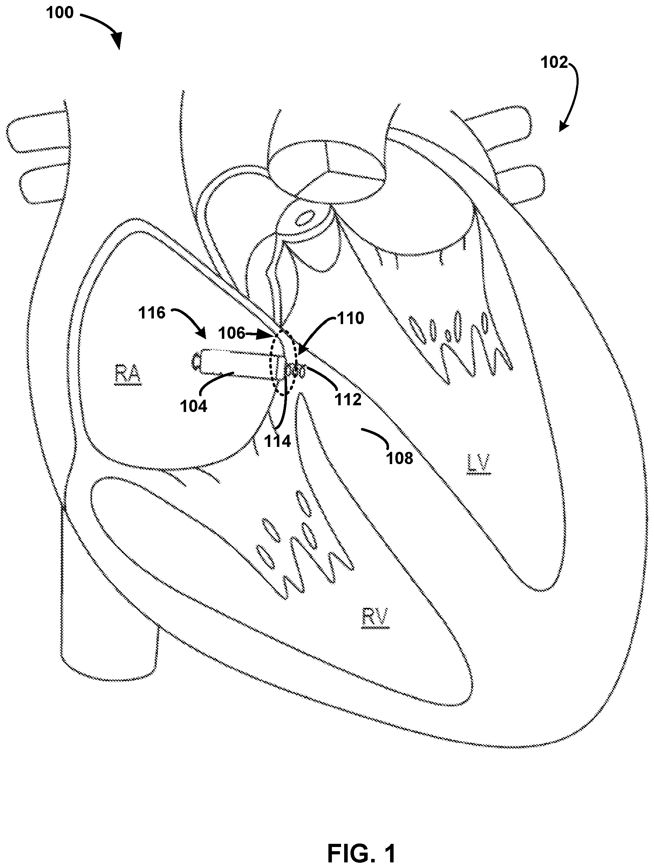

FIG. 1 is a conceptual diagram illustrating an example device 104 implanted in the heart 102 of a patient, in accordance with one or more aspects of this disclosure. Device 104 is shown implanted in the right atrium (RA) of the patient's heart 102 in a target implant region 106, such as the triangle of Koch, in heart 102 of the patient with a distal end of device 104 directed toward the left ventricle (LV) of the patient's heart 102. Although in the example of FIG. 1 the distal end of device 104 is directed toward the LV, the distal end may be directed to other targets, such as interventricular septum of heart 102. Target implant region 106 may lie between the bundle of His and the coronary sinus and may be adjacent the tricuspid valve.

Device 104 includes a distal end 110 and a proximal end 116. Distal end 110 includes a first electrode 112, and a second electrode 114. First electrode 112 may define a helical shape, e.g., as illustrated in FIG. 1. First electrode 112 extends from distal end 110 and may penetrate through the wall tissue of a first chamber (e.g., the RA in the illustrated example) into wall tissue of a second chamber (e.g., ventricular myocardium 108 of the LV in the illustrated example). Second electrode 114 may be disposed on a ramp extending distally from distal end 110 and is configured to be placed in contact with the wall tissue of the first chamber without penetration of the wall tissue of the first chamber by second electrode 114. Second electrode 114 may contact the wall tissue of the first chamber as first electrode 112 penetrates the wall tissue of the first chamber.

The configuration of electrodes 112 and 114 illustrated in FIG. 1 allows device 104 to sense cardiac signals and/or deliver cardiac pacing to multiple chambers of heart 102, e.g., the RA and ventricle(s) in the illustrated example. In this manner, the configuration of electrodes 112 and 114 may facilitate the delivery of A-V synchronous pacing by single device 104 implanted within the single chamber, e.g., the RA. While device 104 is implanted at target implant region 106 to sense in and/or pace the RA and ventricle(s) in the example shown in FIG. 1, a device having an electrode configuration in accordance with the examples of this disclosure may be implanted at any of a variety of locations to sense in and/or pace any one, two or more chambers of heart 102. For example, device 104 may be implanted at region 106 or another region, and first electrode 112 may extend into tissue, e.g., myocardial tissue, of the LV or interventricular septum to, for example, facilitate the delivery of A-V synchronous pacing. Furthermore, a device having an electrode configuration in accordance with the examples of this disclosure may be implanted at any of a variety of locations within a patient for sensing and/or delivery of therapy to other patient tissue. In some examples first electrode 112 may extend into the tissue of heart 102 at region 106 and affix device 104 to the tissue of heart 102.

Additionally, the anti-rotation features and/or functionality described herein are described primarily in the context of a cardiac pacemaker configured to be implanted in one chamber and deliver pacing and sense in that chamber and an additional chamber. However, the anti-rotation features and/or functionality described herein may be included on any implantable medical device, such as an implantable stimulator or implantable lead configured to be fixed at any location or tissue of the body. For example, an implantable stimulator or implantable lead may include one or more ramps on a distal end, the one or more ramps being configured to prevent or inhibit unintended rotation and/or dislodgement of the implantable stimulator or implantable lead from tissue of the patient.

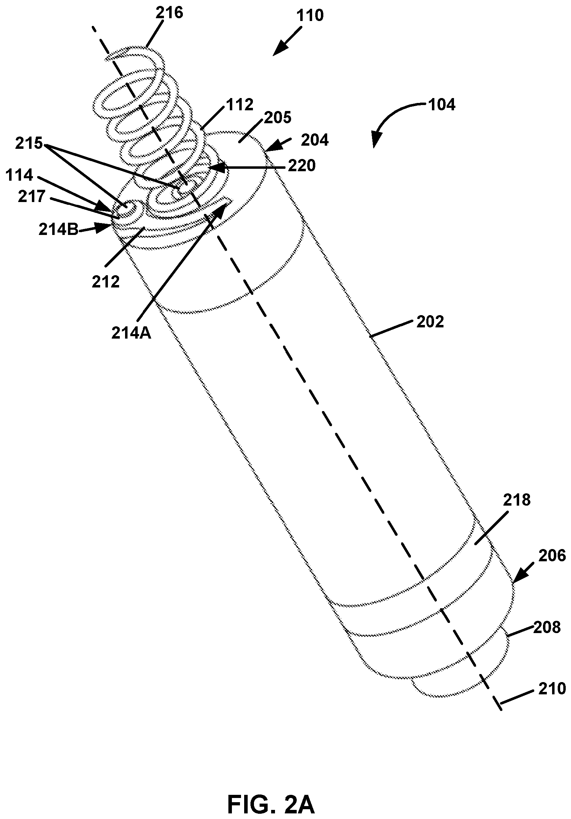

FIG. 2A is a perspective diagram illustrating device 104. Device 104 includes a housing 202 that defines a hermetically sealed internal cavity. Housing 202 may be formed from a conductive material including titanium or titanium alloy, stainless steel, MP35N (a non-magnetic nickel-cobalt-chromium-molybdenum alloy), platinum alloy or other bio-compatible metal or metal alloy, or other suitable conductive material. In some examples, housing 202 is formed from a non-conductive material including ceramic, glass, sapphire, silicone, polyurethane, epoxy, acetyl co-polymer plastics, polyether ether ketone (PEEK), a liquid crystal polymer, other biocompatible polymer, or other suitable non-conductive material.

Housing 202 extends between distal end 204 and proximal end 206 along longitudinal axis 210. In some examples, housing can be cylindrical or substantially cylindrical but may be other shapes, e.g., prismatic, or other geometric shapes. Housing 202 may include a delivery tool interface member 208, e.g., at proximal end 206, for engaging with a delivery tool during implantation of device 104. At distal end 204, housing 202 may define a face 205 of housing 202. Face 205 may define a distal end major surface. Face 205 may be orthogonal to longitudinal axis 210. In some examples, face 205 may be slanted, e.g., face 205 may define a reference plane that is not orthogonal to longitudinal axis 210.

Device 104 includes a ramp 212. Ramp 212 extends from a first end 214A that is fixedly attached to housing 202 at or near distal end 204 (e.g., attached to face 205), to a second end 214B that is more distal to first end 214A. Ramp 212 may be disposed radially outwards of first electrode 112 relative to longitudinal axis 210. Ramp 212 may extend around at least a portion of a perimeter of housing 202. Ramp 212 may extend up to 180 degrees around longitudinal axis 210 and along the perimeter of housing 202. Ramp 212 may be integrally formed as a part of the manufacturing of at least a portion of housing 202 (e.g., as a part of the manufacturing of a header defining distal end 204 and face 205 of housing 202). Ramp 212 may be formed via a molding process, via additive manufacturing, or the like. In some examples ramp 212 is formed separately and affixed to face 205 of housing 202 afterwards. Ramp 212 may define a partial helix, e.g., wound in a same direction and/or in different directions as a helix and/or coil defined by first electrode 112.

Ramp 212 may define a gradient (i.e., "slope") from first end 214A to second end 214B. In some examples, ramp 212 may define a linear gradient from first end 214A to second end 214B. For example, ramp 212 may define a steeper slope first end 214A than at second end 214B, or vice versa.

Ramp 212 may be formed at least partially of an electrically conductive material, such as titanium, platinum, iridium, tantalum, or alloys thereof, and/or of electrically nonconductive material(s). At least portions of ramp 212 may be coated with an electrically insulating coating, e.g., a parylene, polyurethane, silicone, epoxy, or other insulating coating.

Ramp 212 may be an anti-rotation feature. Ramp 212 may increase compression of the tissue and/or increase the friction or other fixation force between the tissue and device 104 and/or first electrode 112. The increase in fixation force(s) may be sufficient to resist rotation of first electrode 112 by movement of the tissue of heart 102 but may not be sufficient to resist rotation of first electrode 112 by the clinician, e.g., to remove device 104 from heart 102. The amount of force the tissue exerts on first electrode 112 and/or the amount of force ramp 212 exerts on the tissue may vary based on movement of heart 102, movement of device 104, movement of fluid within heart 102, size of heart 102, a number of ramp(s) 212 on face 205, presence of additional anti-rotation feature(s), or the like.

In some examples, second end 214B of ramp 212 defines a step extending distally from face 205. The step may require the tissue of heart 102 to deform around and/or over ramp 212 to cause device 104 to rotate, thereby increasing an amount of force required to cause device 104 to rotate.

In some examples, ramp 212 defines a constant width from first end 214A to second end 214B. In some examples, ramp 212 defines a variable width between first end 214A and second end 214B. For example, ramp 212 may define an increasing width from first end 214A to second end 214B. In some examples, a maximum width of ramp 212 is a distance between an outer diameter of housing 202 and an outer diameter of the helix and/or coil defined by first electrode 112. An outer edge (e.g., relative to longitudinal axis 210) of ramp 212 may be coincidental to an outer surface of housing 202. Second end 214B may be separated from face 205 along longitudinal axis by a fixed distance.

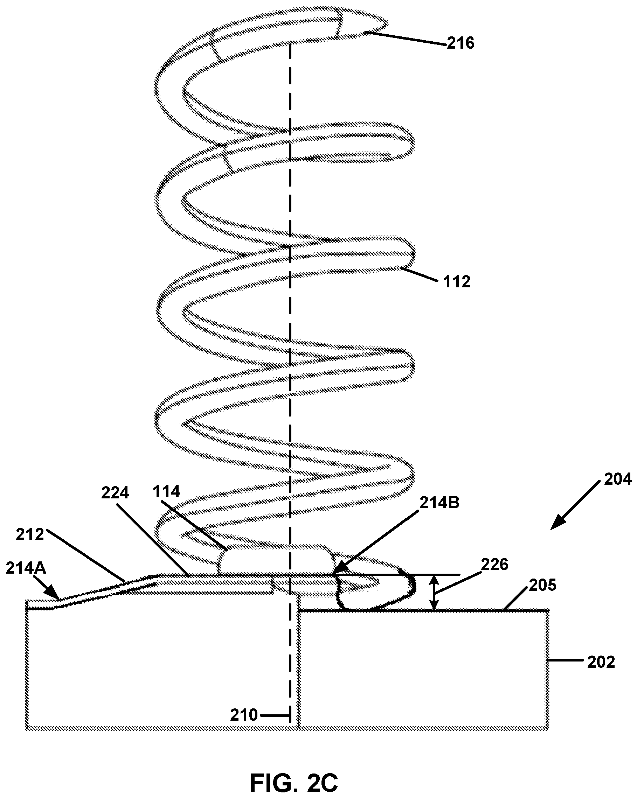

Second end 214B may define a distal surface orthogonal to longitudinal axis 210. Second electrode 114 may be disposed on the distal surface of second end 214B. In some examples, second electrode 114 may be disposed partially along ramp 212, e.g., between first end 214A and second end 214B. In some examples, second electrode 114 is disposed at the base of ramp 212, e.g., on face 205. Ramp 212 may separate second electrode 114 from face 205 by the fixed distance, e.g., to reduce pacing threshold of second electrode 114 and improve contact between second electrode 114 and wall tissue of the first chamber of heart 102. Ramp 212 may be disposed on face 205 and oriented relative to first electrode 112 to place second electrode 114 and/or second end 214B at particular positions around face 205 relative to first electrode 112, as will be described in greater detail with respect to FIG. 2C. In some examples, second electrode 114 may be disposed on distal face 205 or on a proximal portion of ramp 212 (e.g., at first end 214A).

Ramp 212 may provide several advantages over another anti-rotation feature (e.g., a deformable spring electrode). Ramp 212 may prevent or inhibit unintended rotation of device 104 without penetrating the tissue of patient 102. Ramp 212 may simplify the manufacturing process and electrical connections with circuitry within housing 202. For example, ramp 212 may be manufactured as a part of a header of housing 202 instead of being assembled separately after the manufacture process. Ramp 212 may also provide increased surface area compared to a deformable spring electrode, thereby increasing an amount of force ramp 212 may resist prior to rotation of device 104. Additionally, the increased surface area of ramp 212 decreases a likelihood of unintended puncture and/or penetration of the tissue by second electrode 114 and/or by the anti-rotation.

First electrode 112 may include one or more coatings (e.g., electrically insulative coating(s)) configured to define a first electrically active region 216, or first electrode 112 may otherwise define first electrically active region 216. In some examples, first electrically active region 216 may be more proximate to the second, e.g., distal, end of first electrode 112. In the example of FIG. 2A, first electrically active region 216 includes the distal end of electrode 112. Second electrode 114 may include one or more coatings configured to define a second electrically active region 217 on an outer surface of electrode 114. In some examples, as illustrated in FIG. 2A, second electrical active region 217 forms a ring around a therapeutic substance dispensing device 215. Second electrode 114 may include, but is not limited to, may be a button electrode, a spring electrode, or any other suitable type or shape of electrode.

First and second electrodes 112 and 114 may be formed of an electrically conductive material, such as titanium, platinum, iridium, tantalum, stainless steel or alloys thereof. First and second electrodes 112 and 114 may be coated with an electrically insulating coating, e.g., a parylene, polyurethane, silicone, epoxy, or other insulating coating, to reduce the electrically conductive active surface area of first and second electrodes 112 and 114, and thereby define first and second electrically active regions 216 and 217. Defining first and second electrically active regions 216 and 217 by covering portions with an insulating coating may increase the electrical impedance of first and second electrodes 112 and 114 and thereby reduce the current delivered during a pacing pulse that captures the cardiac tissue. A lower current drain conserves the power source, e.g., one or more rechargeable or non-rechargeable batteries, of device 104.

In some examples, first and second electrodes 112 and 114 may have an electrically conducting material coating on first and second electrically active regions 216 and 217 to define the active regions. For example, first and second electrically active regions 216 and 217 may be coated with titanium nitride (TiN). First and second electrodes 112 and 114 may be made of substantially similar material or may be made of different material from one another.

In the example of FIG. 2A, first electrode 112 takes the form of a helix or a coil. First electrode 112 may be an elongated body defining a helix. In some examples, a helix is an object having a three-dimensional shape like that of a wire wound uniformly in a single layer around a cylindrical or conical surface or mandrel such that the wire would be in a straight line if the surface were unrolled into a plane. First electrode 112 may extend from face 205 from proximal end 220 to a distal end, e.g., defining first electrically active region 216. Proximal end 220 may be a location along first electrode 112 where first electrode 112 extends distally past distal end 204 of device 104.

Second electrode 114 is disposed on distal end 204 and may include a button electrode, e.g., as illustrated in FIG. 2A, or any other suitable type or shape of electrode. In some examples, device 104 may have a plurality of second electrodes 114 (e.g., two or more second electrodes 114) disposed on distal end 204 of housing 202. The plurality of second electrodes 114 may be equally spaced around a circumference of distal end 204. At least one of the plurality of second electrodes 114 may be disposed on ramps (e.g., on two or more ramps 212). In some examples, each of the plurality of second electrodes 114 may be disposed on ramps. Each ramp 212 may include a single second electrode 114 or two or more second electrodes 114.In some examples, second electrode 114 may be disposed at a predetermined angle away from first end of first electrode 112.

In some examples, first electrode 112 may include one or more additional anti-rotation features. The additional anti-rotation features may include a shape of first electrode 112, dimensions (e.g., outer diameter, pitch, or the like) of first electrode 112, one or more features disposed on an outer surface of first electrode 112, or the like. The shape and/or dimensions of first electrode 112 may include a geometric shape of first electrode 112, a varying diameter configuration of first electrode 112, a varying pitch configuration of first electrode 112, a waveform configuration of first electrode 112, or any combination herein. The one or more anti-rotation features disposed on first electrode 112 may include, but are not limited to, elongate darts, barbs, or tines. In some examples, the anti-rotation features include bumps, ridges, and/or other texturing disposed on one or more surfaces of ramp 212 and/or of face 205. The one or more anti-rotation features may resist rotation of first electrode 112 (e.g., by penetrating the tissue, by increasing the friction between first electrode 112 and the tissue, or the like) alone or in conjunction with other anti-rotation features (e.g., ramp 212).

As illustrated in FIG. 2A, first electrode 112 may be a helix extending distally from face 205 and revolving around longitudinal axis 210 in a counter-clockwise direction (i.e., "wound" in a counter-clockwise direction, and ramp 212 may define partial helix extending distally from face 205 and revolving around longitudinal axis 210 in a clockwise direction, although in other examples the first electrode 112 and ramp 212 may revolve around longitudinal axis 210 in different directions (e.g., first electrode 112 revolves around longitudinal axis 210 in a clockwise direction and ramp 212 revolves around longitudinal axis 210 in a counter-clockwise direction) or first electrode 112 and ramp 212 may revolve around longitudinal axis 210 in a same direction. Designing ramp 212 to revolve around longitudinal axis 210 in the same direction as first electrode 112 may increase the resistance to insertion and reduce the resistance to removal of device 104 by the clinician while increasing the resistance to rotation of device 104 by movement of the tissue and/or movement of heart 102. In some examples, first electrode 112 and ramp 212 may revolve around longitudinal axis 210 in different directions. In such examples, designing first electrode 112 and ramp 212 to revolve around longitudinal axis 210 in different directions may reduce the resistance to insertion and increase the resistance to removal of device 104 by the clinician. In the example of FIG. 2A, the helix and partial helix defined by first electrode 112 and ramp 212, respectively, have the same pitch, although they may have different pitches in other examples. In some examples, first electrode 112 defines a varying pitch along longitudinal axis 210. In some examples, one or both of first electrode 112 and ramp 212 may define a shape other than helical. For example, first electrode 112 may define a geometrical shape (e.g., a triangular shape, a rectangular shape, a hexagonal shape, an octagonal shape, a lobed shape, or the like). Such a geometrical shape may be equilateral. The geometrical shape may function as an anti-rotation feature.

First and second electrodes 112 and 114 may vary in size and shape in order to enhance tissue contact of first and second electrically active regions 216 and 217. For example, first electrodes 112 may have a round cross-section or could be made with a flatter cross-section (e.g., oval or rectangular) based on tissue contact specifications. In some examples, second electrode 114 may have an outer surface that varies in size and shape (e.g., an oval outer surface, an outer surface with a larger diameter, or the like) in order to enhance tissue contact of second electrically active region 217.

The size and shape of first electrode 112 may be determined at least in part by stiffness requirements. For example, stiffness requirements may vary based on the expected implantation requirements, including the tissue into which the electrodes are implanted or contact, as well as how long device 104 is intended to be implanted.

The distal end of first electrode 112 can have a conical, hemi-spherical, or slanted edge distal tip with a narrow tip diameter, e.g., less than 1 millimeter (mm), for penetrating into and through tissue layers. In some examples, the distal end of first electrode can be a sharpened or angular tip or sharpened or beveled edges, but the degree of sharpness may be constrained to avoid a cutting action that could lead to lateral displacement of the distal end of first electrode 112 and undesired tissue trauma. In some examples, first electrode 112 may have a maximum diameter at its base that interfaces with housing distal end 204. In such examples, the outer diameter of the helix defined by first electrode 112 may decrease from housing distal end 204 to the distal end of first electrode 112. In some examples, the diameter of first electrode 112 may vary from housing distal end 204 to the distal end of first electrode 112. The varying diameter may cause first electrode 112 to resist rotation within the tissue of heart 102.

The outer dimensions of first electrode 112 can be substantially straight and cylindrical, with first electrode 112 being rigid in some examples. In some examples, first electrode 112 may have flexibility in lateral directions, being non-rigid to allow some flexing with heart motion. In a relaxed state, when not subjected to any external forces, first electrode 112 can be configured to maintain a distance between first electrically active region 216 and housing distal end 204.

Distal end of first electrode 112 can pierce through one or more tissue layers to position first electrically active region 216 within a desired tissue layer, e.g., the ventricular myocardium 108 or interventricular septum. Accordingly, first electrode 112 extends a distance from housing distal end 204 corresponding to the expected pacing site depth and may have a relatively high compressive strength along its longitudinal axis, which may be substantially similar to or coincident with longitudinal axis 210, to resist bending in a lateral or radial direction when a longitudinal, axial, and/or rotational force is applied, e.g., to the proximal end 206 of housing 202 to advance device 104 into the tissue at target implant region 106. By resisting bending in a lateral or radial direction, first electrode 112 can maintain a spacing between a plurality of windings of first electrode 112 when first electrode 112 is a helix electrode. The spacing may be a pre-determined pitch of first electrode 112 and may vary from distal end 204 to the distal end of first electrode 112. First electrode 112 may be longitudinally non-compressible. First electrode 112 may also be elastically deformable in lateral or radial directions when subjected to lateral or radial forces, however, to allow temporary flexing, e.g., with tissue motion, but returns to its normally straight position when lateral forces diminish. In some examples, when first electrode 112 is not exposed to any external force, or to only a force along its longitudinal axis (substantially similar to or coincident with longitudinal axis 210), first electrode 112 retains a straight, linear configuration as shown.

All, substantially all, or a portion of housing 202 may function as an electrode 218, e.g., an anode, during pacing and/or sensing. In some examples, electrode 218 can circumscribe a portion of housing 202 at or near proximal end 206. Electrode 218 can fully or partially circumscribe housing 202. FIG. 2A shows electrode 218 extending as a singular band. Electrode 218 can also include multiple segments spaced a distance apart along a longitudinal axis 210 of housing 202 and/or around a perimeter of housing 202. In some examples, electrode 218 may be disposed on face 205 or on another ramp 212 disposed on face 205. For example, electrode 114 may be disposed on a first ramp 212 and electrode 218 may be disposed on a second ramp 212.

When housing 202 is formed from a conductive material, such as a titanium alloy, portions of housing 202 may be electrically insulated by a non-conductive material, such as a coating of parylene, polyurethane, silicone, epoxy or other biocompatible polymer, or other suitable material. For the portions of housing 202 without the non-conductive material, one or more discrete areas of housing 202 with conductive material can be exposed to define electrode 218.

When housing 202 is formed from a non-conductive material, such as a ceramic, glass or polymer material, an electrically-conductive coating or layer, such as a titanium, platinum, stainless steel, alloys thereof, a conductive material may be applied to one or more discrete areas of housing 202 to form electrode 218.

In some examples, electrode 218 may be a component, such as a ring electrode, that is mounted or assembled onto housing 202. Electrode 218 may be electrically coupled to internal circuitry of device 104 via electrically-conductive housing 202 or an electrical conductor when housing 202 is a non-conductive material. In some examples, electrode 218 is located proximate to proximal end 206 of housing 202 and can be referred to as a proximal housing-based electrode. Electrode 218 can also be located at other positions along housing 202, e.g., located proximately to distal end 204 or at other positions along longitudinal axis 210.

In some examples, second electrode 114 or electrode 218 may be paired with first electrode 112 for sensing ventricular signals and delivering ventricular pacing pulses. In some examples, second electrode 114 may be paired with electrode 218 or first electrode 112 for sensing atrial signals and delivering pacing pulses to atrial tissue (e.g., to the atrial myocardium) in target implant region 106. In other words, electrode 218 may be paired, at different times, with first electrode 112 and/or second electrode 114 for either ventricular or atrial functionality, respectively, in some examples. In some examples, first and second electrodes 112 and 114 may be paired with each other, with different polarities, for atrial and ventricular functionality.

In some examples, second electrode 114 may be configured as an atrial cathode electrode for delivering pacing pulses to the atrial tissue, e.g., at target implant region 106 in combination with electrode 218. Second electrode 114 and electrode 218 may also be used to sense atrial P-waves for use in controlling atrial pacing pulses (delivered in the absence of a sensed P-wave) and for controlling atrial-synchronized ventricular pacing pulses delivered using first electrode 112 as a cathode and electrode 218 as the return anode.

A distal end of first electrode 112 can be configured to rest within a ventricular myocardium of the patient, and second electrode 114 and ramp 212 can be configured to contact an atrial endocardium of the patient. Device 104 may include more or fewer electrodes than two electrodes. In some examples, device 104 may include one or more second electrodes 114 along housing distal end 204. For example, device 104 may include two or three electrodes configured for atrial functionality like second electrode 114, and the three electrodes may be substantially similar or different from one another. Spacing between a plurality of second electrodes 114 may be at an equal or unequal distance. Second electrode(s) 114 may be individually selectively coupled to sensing and/or pacing circuitry enclosed by housing 202 for use as an anode with first electrode 112 or as an atrial cathode electrode, or may be electrically common and not individually selectable. In some examples, in place of first electrode 112, device 104 may include a fixation element (not shown) of similar shape and mechanical, but without an electrically active region or electrode formed thereon or borne thereby; in such examples, electrically active region 216 can be positioned on a separate member and/or on the housing 202.

Inflammation of patient tissue may result from interaction with device 104. For example, penetration of tissue by first electrode 112 and/or contact between tissue and second electrode 114 may result in inflammation of the tissue. Inflammation of patient tissue proximate to first and second electrodes 112 and 114 may result in higher thresholds for stimulation delivered to the tissue to activate, or capture, the tissue. Higher capture thresholds may, in turn, increase the consumption of a power source of device 104 associated with delivery of the stimulation.

In some examples device 104 includes one or more therapeutic substance dispensing devices 215, e.g., on face 205, within a recess defined by second electrode 114, on ramp 212. The steroid may mitigate inflammation of patient tissue resulting from interaction with the IMD. Therapeutic substance dispensing devices 215 may be configured to elute one or more steroids to tissue in proximity to therapeutic substance dispensing devices 215 over time. In some examples, steroid eluting elements 215 comprise one or more monolithic controlled release devices (MCRDs).

In some examples, device 104 includes one or more therapeutic substance dispensing devices 215 configured to elute one or more steroids to tissue proximate to first electrode 112. Therapeutic substance dispensing devices 215 may be disposed within a recess defined by second electrode 114. In some examples, therapeutic substance dispensing devices 215 may be disposed at a center of face 205, e.g., within recess defined by housing 202, and/or on ramp 212, e.g., between first end 214A and second end 214B.

Ramp 212 may cause second electrode 114 to maintain consistent contact with the wall tissue, e.g., by raising second electrode 114 from face 205 by a fixed distance. Consistent contact between second electrode 114 and the wall tissue may improve electrical conductivity and the delivery of electrical signals from second electrode 114 to the wall tissue. In some examples, where device 104 is an implantable pacing device, the consistent contact between second electrode 114 and the wall tissue may reduce and/or maintain a pacing threshold for a chamber (e.g., the right atrium) of heart 102.

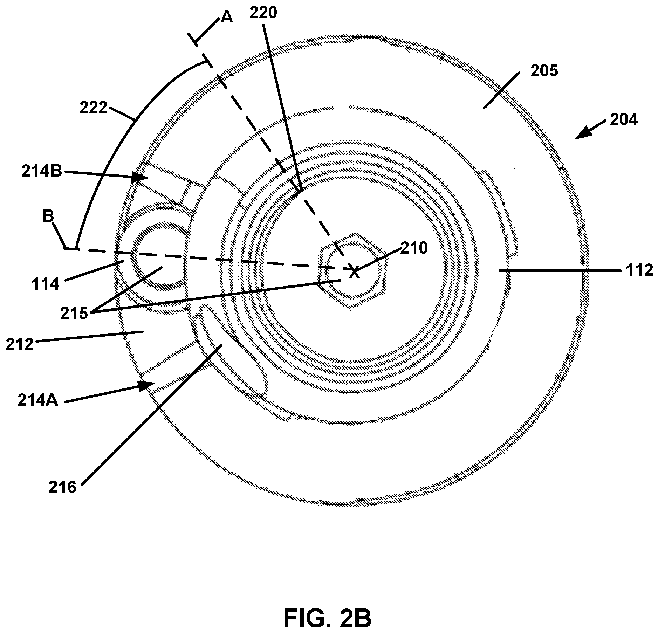

FIG. 2B is a perspective diagram illustrating a top-down view of the example device 104 of FIG. 2A. As illustrated in FIG. 2B, second electrode 114 may be separated from a proximal end 220 of first electrode 112 by a predetermined angle 222. First electrode 112 may extend from a proximal end 220 to a distal end (e.g., at first electrically active region 216). Proximal end 220 may define a first reference axis A with a center of device 104 (e.g., with longitudinal axis 210 of device 104). Second electrode 114 may define a second reference axis B with the center of device 104 (e.g., with longitudinal axis 210 of device 104). First reference axis A and second reference axis B may define angle 222. As such, in some examples the separation of second electrode 114 and first electrode 112 by predetermined angle 222 may be defined as a separation angle between second electrode 114.

In some examples, an implantable device (e.g., device 104) may include one or more prominent features (also referred to herein as "prominences") extending from a distal end major surface of the device (e.g., face 205 of device 104). The prominence may include a ramp (e.g., ramp 212), a distalmost portion of a ramp, a pillar, an extrusion, an extension, an electrode, or the like. Predetermined angle 222, as described herein, may be used to determine placement of the prominence on the distal end major surface of the device and/or relative to a helix (e.g., a helical electrode, a fixation helix) disposed on the distal end major surface.

Face 205 may define a coordinate system (e.g., a polar coordinate system) around longitudinal axis 210. For ease of reference, predetermined angle 222, and/or placement of ramp 212, first electrode 112, and/or second electrode 114 is described herein primarily with reference to a polar coordinate system having 0 degrees along first reference axis A. However, any other coordinate system may be used to define predetermined angle 222 and/or the placement of ramp 212, first electrode 112, and/or second electrode 114.

Predetermined angle 222 may be between about 0 degrees to about 90 degrees or between about 180 degrees to about 360 degrees. In some examples, predetermined angle 222 may include locations around the circumference of distal end 204 where a distance between face 205 and first electrode 112 is less than or equal to about 0.5 mm (e.g., about 0.02 in). For example, when second electrode 112 is disposed at predetermined angle 222 from proximal end 220 of first electrode 112, a portion of first electrode 112 separated from proximal end 220 by at least the predetermined angle 222 may be separated from face 205 of device 105 by at least 0.5 mm. Placing second electrode 114 at predetermined angle 222 relative to proximal end 220 of first electrode 112 may improve anti-rotation capabilities of ramp 212 without causing unintended reactions (e.g., unintended interactions between one or more coils of first electrode 112 and second end 214B of ramp 212 which may cause increased pinching of the tissue and/or destabilization of device 104 in the tissue. For example, placement of second electrode 114 at predetermined angle 222 of between 90 and 180 degrees may lead to increased destabilization of device 104. Therefore, predetermined angle 222 of less than or equal to 90 degrees or greater than or equals to 180 degrees may be preferred to improve stability of device 104.

Predetermined angle 222 may be any angle greater than 90 degrees following the helix's complete exit from header 205 Predetermined angle 222 may be adjusted based on the dimensions of first electrode 112 (e.g., outer diameter of first electrode 112, cross-sectional diameter of first electrode 112, pitch of first electrode 112) and/or the dimensions of ramp 212 (e.g., pitch of ramp 212, width of ramp 212, height of ramp 212, surface area of second end 214B of ramp 212). Predetermined angle 222 may optimally be between 180 degrees and 270 degrees, e.g., to balance stabilization of device 104 with electrical isolation capabilities of first electrode 112 and second electrode 114, e.g., to balance stabilization and pacing capabilities of device 104. Predetermined angle 222 may begin at axis A and increase zero while proceeding in a distally advancing wind direction of the helix (e.g., in a same direction as the winding of first electrode 112).

FIG. 2C is a perspective diagram illustrating a side view of the distal end 204 of the example device 104 of FIG. 2A. Ramp 212 defines a distal surface 224. Distal surface 224 may be parallel to face 205 and second electrode 114 may be disposed on distal surface 224. Second end 214B of ramp 212 may have a height (e.g., away from face 205) of a distance 226 (also referred to herein as "fixed distance 226"). Distance 226 may be between about 0.5 mm and about 4 mm. In some examples, a combined height of second end 214B and second electrode 114 along longitudinal axis 210 is between about 0.5 mm and about 4 mm.

As illustrated in FIG. 2C, second end 214B may extend distally from a distal end major surface of distal end 204 (e.g., face 205), e.g., towards second electrode 114. Second end 214B may extend from face 205 for a distance 226. Second end 214B may extend distally in a parallel direction as longitudinal axis 210. In some examples, second end 214B forms a right angle with face 205.

In some examples, additional inserts may be disposed on distal surface 224, e.g., to adjust a height of ramp 212 and/or of second electrode 114. Each insert may be a disc, plate, ring, or the like configured to be affixed to ramp 212 and adjust a height and/or an orientation of ramp 212. Second electrode 114 may be disposed on a distalmost insert. Inserts may have angled distal surfaces, e.g., to control an orientation of second electrode 114 relative to face 205 and/or first electrode 112.

Device 104 may include one or more additional anti-rotation features including but are not limited, barbs, protrusions, recesses, textured surfaces, or the like. The anti-rotation features may be disposed on at least a portion of face 205, an outer surface of housing 202, and/or on ramp 212. The anti-rotation features may be disposed on a portion of ramp 212 extending from first end 214A to distal surface 224, on distal surface 224, and/or around an outer surface of ramp 212 surrounding second end 214B.

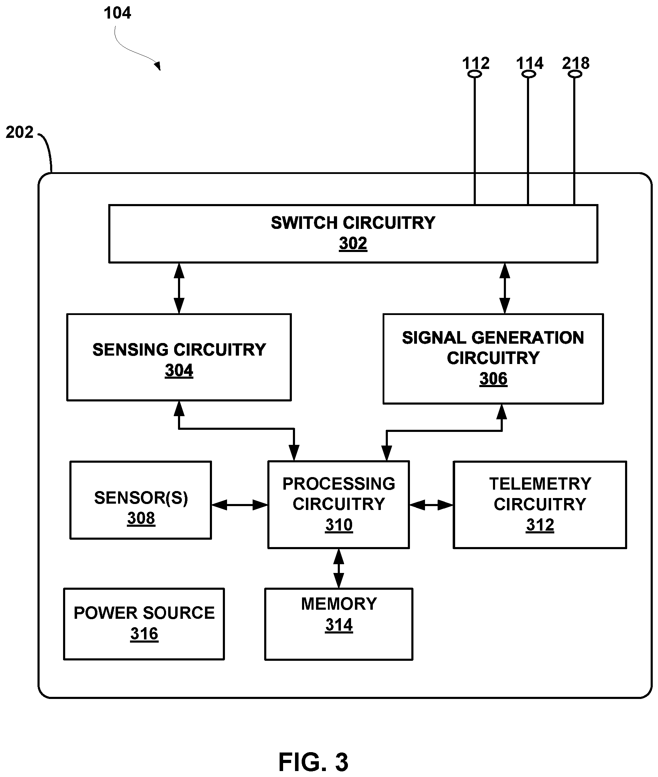

FIG. 3 is a functional block diagram illustrating an example configuration of device 104. As illustrated in FIG. 3, device 104 include electrodes 112 and 114, which may be configured as described with respect to FIGS. 1 and 2. For example, as described with respect to FIGS. 1 and 2, first electrode 112 may be configured to extend from distal end 204 of housing 202 and may penetrate through the wall tissue of a first chamber (e.g., the RA) into wall tissue of a second chamber (e.g., the LV). Second electrode 114 extends from distal end 204 of housing 202 and may be configured to maintain contact with the wall tissue of the first chamber without penetration of the wall tissue of the first chamber by second electrode 114. Second electrode 114 may maintain contact in this manner by virtue of being disposed in and/or on ramp 212 or another ramp as described herein. Ramp 212 may position second electrode 114 at a distance (e.g., distance 226) away from face 205, thereby causing second electrode 114 to maintain consistent contact with the wall tissue when first electrode 112 is secured within the wall tissue.

In the example shown in FIG. 3, device 104 includes switch circuitry 302, sensing circuitry 304, signal generation circuitry 306, sensor(s) 308, processing circuitry 310, telemetry circuitry 312, memory 314, and power source 316. The various circuitry may be, or include, programmable or fixed function circuitry configured to perform the functions attributed to respective circuitry. Memory 314 may store computer-readable instructions that, when executed by processing circuitry 310, cause device 104 to perform various functions. Memory 314 may be a storage device or other non-transitory medium. The components of device 104 illustrated in FIG. 3 may be housed within housing 202.

Signal generation circuitry 306 generates electrical stimulation signals, e.g., cardiac pacing pulses. Switch circuitry 302 is coupled to electrodes 112, 114, and 218, may include one or more switch arrays, one or more multiplexers, one or more switches (e.g., a switch matrix or other collection of switches), one or more transistors, or other electrical circuitry. Switch circuitry 302 is configured to direct stimulation signals from signal generation circuitry 306 to a selected combination of electrodes 112, 114, and 218, having selected polarities, e.g., to selectively deliver pacing pulses to the RA, ventricles, or interventricular septum of heart 102. For example, in order to pace one or both of the ventricles, switch circuitry 302 may couple first electrode 112, which has penetrated to wall tissue of a ventricle or the intraventricular septum, to signal generation circuitry 306 as a cathode, and one or both of second electrode 114 or electrode 218 to signal generation circuitry 306 as an anode. As another example, in order to pace the RA, switch circuitry 302 may couple second electrode 114, which maintains contact with the RA endocardium, to signal generation circuitry 306 as a cathode, and one or both of first electrode 112 or electrode 218 to signal generation circuitry 306 as an anode.

Each of electrodes 112, 114, 218 may be coupled to switch circuitry 302 via a corresponding feedthrough assembly. In some examples, each feedthrough assembly may be substantially straight (e.g., along longitudinal axis 210). In some examples, such as when distal end 204 of housing 202 is removable from housing 202 (e.g., when distal end 204 is a removable header), the feedthrough assemblies may be offset to allow for removal of distal end 204. For example, when a header defining distal end 204 is configured to be removably secured to housing 202 (e.g., via a turn-lock mechanism), the feedthrough assemblies may be offset from longitudinal axis 210 to allow the header to turn relative to housing 202.

Switch circuitry 302 may also selectively couple sensing circuitry 304 to selected combinations of electrodes 112, 114, and 218, e.g., to selectively sense the electrical activity of either the RA or ventricles of heart 102. Sensing circuitry 304 may include filters, amplifiers, analog-to-digital converters, or other circuitry configured to sense cardiac electrical signals via electrodes 112, 114, and/or 218. For example, switch circuitry 302 may couple each of first electrode 112 and second electrode 114 (in combination with electrode 218) to respective sensing channels provided by sensing circuitry 304 to respectively sense either ventricular or atrial cardiac electrical signals. In some examples, sensing circuitry 304 is configured to detect events, e.g., depolarizations, within the cardiac electrical signals, and provide indications thereof to processing circuitry 310. In this manner, processing circuitry 310 may determine the timing of atrial and ventricular depolarizations, and control the delivery of cardiac pacing, e.g., AV synchronized cardiac pacing, based thereon. Processing circuitry 310 may include any one or more of a microprocessor, a controller, a digital signal processor (DSP), an application specific integrated circuit (ASIC), a field-programmable gate array (FPGA), discrete logic circuitry, or any other processing circuitry configured to provide the functions attributed to processing circuitry 310 herein may be embodied as firmware, hardware, software or any combination thereof.

Sensor(s) 308 may include one or more sensing elements that transduce patient physiological activity to an electrical signal to sense values of a respective patient parameter. Sensor(s) 308 may include one or more accelerometers, optical sensors, chemical sensors, temperature sensors, pressure sensors, or any other types of sensors. Sensor(s) 308 may output patient parameter values that may be used as feedback to control sensing and delivery of therapy by device 104.

Telemetry circuitry 312 supports wireless communication between device 104 and an external programmer (not shown in FIG. 3) or another computing device under the control of processing circuitry 310. Processing circuitry 310 of device 104 may receive, as updates to operational parameters from the computing device, and provide collected data, e.g., sensed heart activity or other patient parameters, via telemetry circuitry 312. Telemetry circuitry 312 may accomplish communication by radiofrequency (RF) communication techniques, e.g., via an antenna (not shown).

Power source 316 delivers operating power to various components of device 104. Power source 316 may include a rechargeable or non-rechargeable battery and a power generation circuit to produce the operating power. Recharging may be accomplished through proximal inductive interaction between an external charger and an inductive charging coil within device 104.

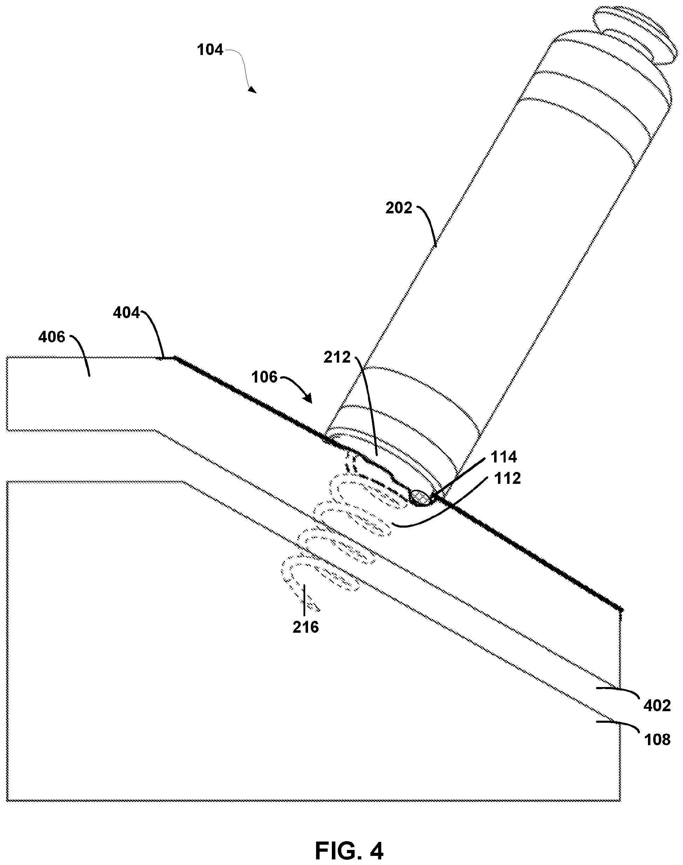

FIG. 4 is a conceptual diagram of device 104 implanted at target implant region 106. First electrode 112 may be inserted (e.g., in a manner similar to rotating and advancing a threaded screw) such that tissue becomes engaged with the helix of first electrode 112. As first electrode 112 becomes engaged with tissue, first electrode 112 pierces into the tissue at target implant region 106 and advances through atrial myocardium 406 and central fibrous body 402 to position first electrically active region 216 in ventricular myocardium 108 as shown in FIG. 4. In some examples, first electrode 112 penetrates into the interventricular septum. In some examples, first electrode 112 does not perforate either of the ventricular endocardial or epicardial surface.

In some examples, manual pressure applied to the housing proximal end 206, e.g., via an advancement tool, provides the longitudinal force to pierce the cardiac tissue at target implant region 106. In some examples, actuation of an advancement tool rotates device 104 and first electrode 112 configured as a helix about longitudinal axis 210. The rotation of the helix about the longitudinal axis 210 advances first electrode 112 through atrial myocardium 406 and central fibrous body 402 to position first electrically active region 216 in ventricular myocardium 108 as shown in FIG. 4.

As first electrode 112 advances into the tissue, the distance between second electrode 114 (e.g., on ramp 212) and atrial endocardium 404 decreases until second electrode 114 and ramp 212 contact, and may press against, the surface of atrial endocardium 404. Second electrode 114 and ramp 212 may press against the surface of atrial endocardium 404 and compress the wall tissue. The compression of the wall tissue may increase friction between ramp 212 and the wall tissue and prevent or inhibit rotation of device 104 due to movement of tissue of heart 102 (e.g., movement of ventricular myocardium 108, atrial myocardium 406, central fibrous body 402, or the like) or blood flow during cardiac function. Ramp 212 pressing against heart tissue may cause heart tissue to become engaged with second electrically active region 217 of second electrode 114 disposed on ramp 212. Second electrode 114 is held in contact with atrial endocardium 404 by first electrode 112 and ramp 212. Retraction of second electrode 114 from the surface of atrial endocardium 404 may be prevented or inhibited by first electrode 112 and ramp 212.

Ramp 212 can be the sole anti-rotation feature of device 104 in some examples. In some examples, device 104 may have one or more additional anti-rotation features, e.g., defined and/or disposed on first electrode 112, on face 205, and/or on ramp 212. The distance by which first electrode 112 extends from housing 202 can be selected so first electrically active region 216 reaches an appropriate depth in the tissue layers to reach the targeted pacing and sensing site, in this case in ventricular myocardium 108, without puncturing all the way through into an adjacent cardiac chamber.

Target implant region 106 in some pacing applications is along atrial endocardium 404, substantially inferior to the AV node and bundle of His. First electrode 112 can have a length that penetrates through atrial endocardium 404 in target implant region 106, through the central fibrous body 402 and into ventricular myocardium 108 without perforating through the ventricular endocardial surface. In some examples, when the full length of first electrode 112 is fully advanced into target implant region 106, first electrically active region 216 rests within ventricular myocardium 108 and second electrode 114 is positioned in intimate contact with atrial endocardium 404. First electrode 112 may extend from housing distal end 204 approximately 3 mm to 12 mm in various examples. In some examples, first electrode 112 may extend a distance from distal end 204 by at least 3 mm, at least 3 mm but less than 20 mm, less than 15 mm, less than 10 mm, or less than 8 mm in various examples. The diameter of an elongated body defining first electrode 112 may be 2 mm or less, e.g., may be 1 mm or less, may be 0.6 mm or less. An outer diameter of the helix or coil defined by first electrode 112 may be 4 mm or less.

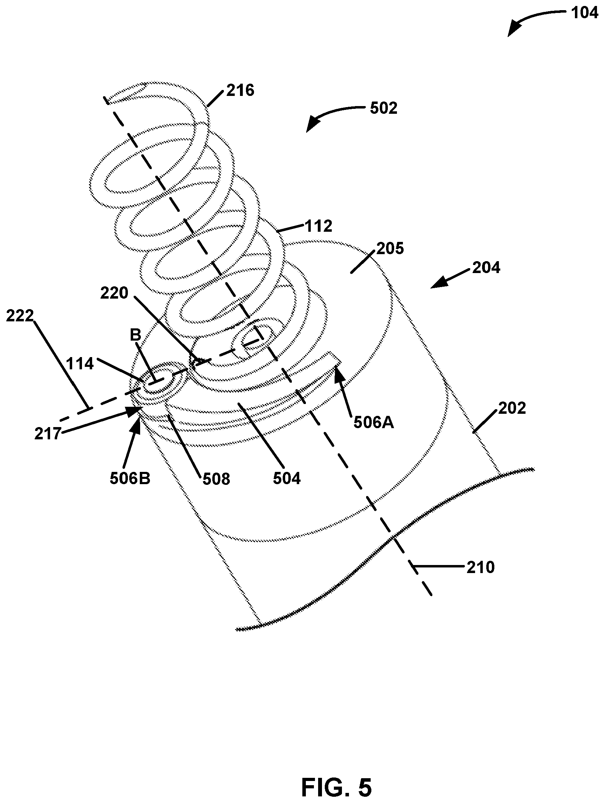

FIG. 5 is a perspective diagram illustrating another example distal end 502 of example device 104 of FIG. 1. Distal end 502 may include a ramp 504 extending from face 205 and defining a recess 508. Second electrode 114 may be disposed within recess 508. First electrode 112, second electrode 114, and housing 202 may be similar to the components of device 104, as described above in FIGS. 2A-2C. Ramp 504 may extend distally from face 205 and along a perimeter of housing 202. Ramp 504 may extend from first end 506A distally to second end 506B, e.g., in a similar manner as ramp 212. Ramp 504 may define recess 508 at second end 506B. A distal surface of second electrode 114 may be coincident with a distalmost surface of ramp 504. Compared to ramp 212 illustrated in FIGS. 2A-2C, ramp 504 with recess 508 reduces a height of second electrode 114 relative to longitudinal axis 210 and increases sensing and/or pacing by second electrode 114 around sides of second electrode 114 (e.g., along planes normal to longitudinal axis 210). Placing second electrode 114 coincident or flush with a distalmost surface of ramp 504 may inhibit second electrode 114 from interfering with rotation of device 104 during implantation and/or extraction of device 104 without reducing anti-rotation capabilities of ramp 504. Placing second electrode 114 coincident to the distalmost surface of ramp 504 may reduce pacing thresholds of second electrode 114, e.g., by causing second electrode 114 to maintain stable contact with tissue of heart 102 despite compression of the tissue by ramp 504. Ramp 504 may be configured to retain one or more inserts (not pictured) within recess 508, e.g., to adjust a depth of recess 508 and thereby an overall height of second electrode 114. In some examples, ramp 504 may be configured to retain one or more inserts within recess 508 such that the distalmost surface of the one or more inserts is coincident with a distalmost surface of ramp 504, e.g., to modify the dimensions and structure of ramp 504 to mimic the structure and function of ramp 212 as previously described herein.

Electrically active region 217 of second electrode 114 may extend around the entire parameter of electrode 114 and/or the entire outer surface of electrode 114. In some examples, electrically active region 217 may extend around the outer surface of electrode 114 and around a portion of the perimeter of electrode 114. For example, as illustrated in FIG 5, electrically active region 217 of second electrode 114 may be a portion of the outer surface of second electrode 114 disposed along the perimeter of housing 202. In some examples, electrically active region 217 encompasses at least a portion of the outer surface of ramp 212 (e.g., along a distalmost surface of ramp 212, e.g., along a radially outermost surface of ramp 212).

Second electrode 114 may define predetermined angle 222, e.g., in accordance with the previous techniques described herein. For example, FIG. 5 illustrates second electrode 114 and proximal end 220 of first electrode 112 being separated by a predetermined angle 222 of 0 degrees.

In some examples, another second electrode 114 may be disposed at a foot of ramp 504, e.g., at first end 506A. In such examples, each second electrode 114 may retain a therapeutic substance dispensing device 215 and may be separated from proximal end 220 by at least predetermined angle 222. Each second electrode 114 may also deliver electrical stimulation and/or sense electrical signals along longitudinal axis 210 while second electrode 114 in recess 508 delivers electrical stimulation and/or senses electrical signals along a reference plane orthogonal to longitudinal axis 210.

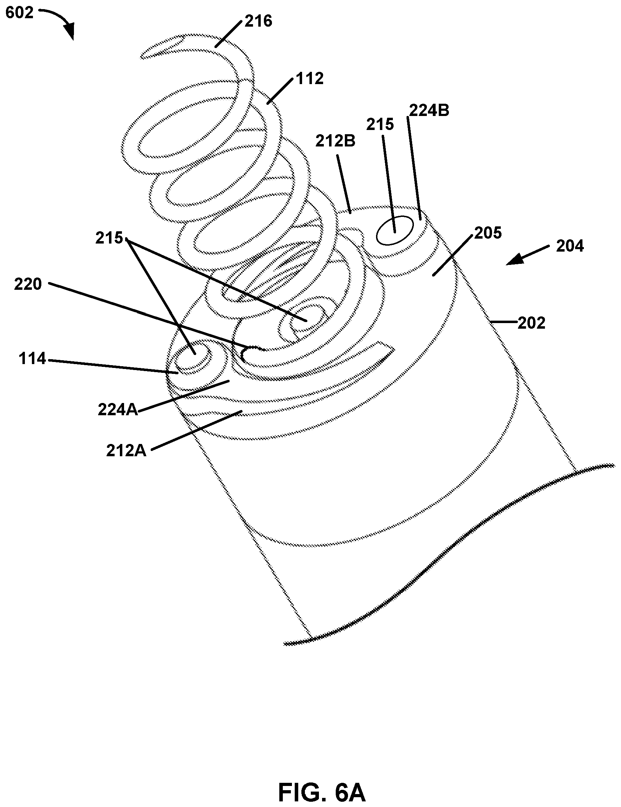

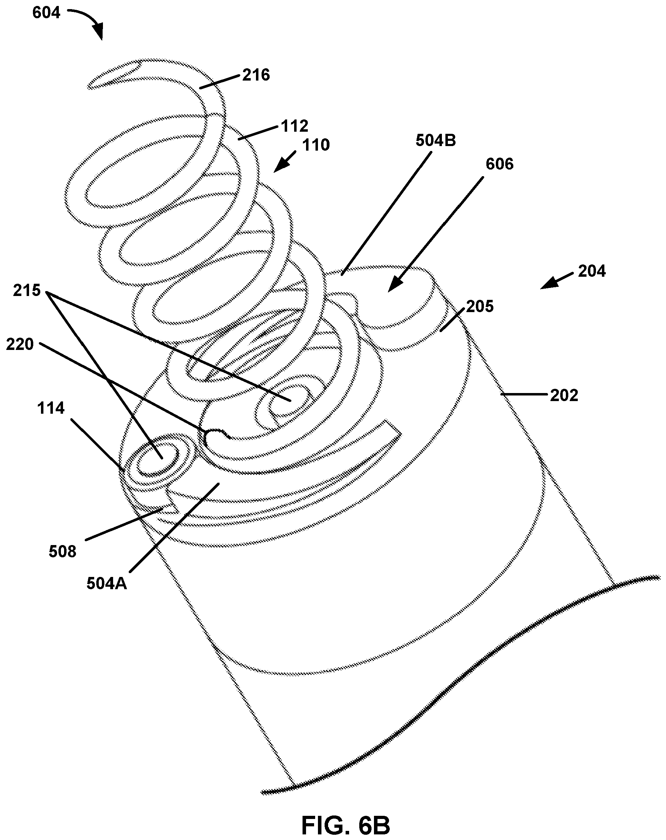

FIG. 6A is a perspective diagram illustrating an example distal end 602 of example device 104 of FIG. 1 with two ramps 212. FIG. 6B is a perspective diagram illustrating an example distal end 604 example device 104 of FIG. 3 with two ramps 504. Aside from the descriptions included below, dimensions, properties, and/or functionalities of any of the components of illustrated in FIGS. 6A and 6B are the same as those previously discussed herein.

FIG. 6A illustrates device 104 having distal end 602 with two ramps 212A, 212B (collectively referred to as "ramps 212") disposed on face 205 of distal end 204 of housing 202. While FIG. 6A illustrated two ramps 212, other example devices may include three or more ramps 212 disposed on face 205. Ramps 212 are arranged radially outward of first electrode 112 and around a perimeter of housing 202. Each of ramps 212 defines a distal surface 224. For example, ramp 212A defines distal surface 224A and ramp 212B defines distal surface 224B (collectively referred to as "distal surfaces 224"). Second electrode 114 may be disposed on a distal surface 224 of one of ramps 212 (e.g., ramp 212A). The other ramp 212 (e.g., ramp 212B) may include a therapeutic substance dispensing device 215 disposed on distal end 224 and may dispense therapeutics (e.g., steroids) to reduce inflammation of the tissue.

In some examples, ramps 212 may have a same height (e.g., a same distance 226 of FIG. 2C). For example, distal surfaces 224 of ramps 212 may have a same height and placement of second electrode 114 causes ramp 212A to be higher than ramp 212B, e.g., by a height of second electrode 114. In some examples, ramps 212 have a same overall height (e.g., including the height of second electrode 224), e.g., to improve stability of device 104 within the tissue. For example, ramp 212A includes second electrode 114 disposed on distal surface 224 and distal surface 224A of ramp 212A is lower than distal surface 224B of ramp 212B but the overall heights of ramp 212A and ramp 212B are the same. In some examples, ramps 212 have different heights to accommodate for a particular orientation of distal end 502 relative to the tissue of heart 102, e.g., as a result of the placement and dimensions of first electrode 112, second electrode 114, and ramps 212 on device 104. For example, ramp 212B is lower or higher than ramp 212A to account for a tilt of device 104 relative to an outer surface of tissue of heart 102 when device 104 is implanted within the tissue.

Additional anti-rotation features may be disposed on one or more of ramps 212 (e.g., on distal surfaces 224, along a surface extending between first end 214A and distal surface 224 of any of ramps 212). The additional anti-rotation features may increase resistance of distal end 502 to unintended rotation, dislodgement, and/or oscillation of device 104, e.g., as a result of the normal functions of heart 102.

FIG. 6B illustrates device 104 having distal end 604 with two ramps 504A, 504B (collectively referred to as "ramps 504") disposed on face 205 of distal end 204 of housing 202. While FIG. 6B illustrated two ramps 504, other example devices may include three of more ramps 504 disposed on face 205.

In some examples, one of ramps 504 (e.g., ramp 504A) may include recess 508 configured to retain second electrode 114 and another of ramps 504 (e.g., ramp 504B) may not include recess 508. Ramp 504B may instead define a distal surface 606, e.g., similar to distal surfaces 224 of ramps 212. A distal surface of second electrode 114 may be higher, lower, or of the same height as distal surface 606, e.g., in accordance with any of the example devices described above with respect to FIG. 6A.

In some examples, ramp 504B may include recess 508 configured to receive one or more inserts. A clinician may adjust the height of either of ramps 504 via insertion or removal of inserts. In such a manner, distal end 604 of device 104 may include ramps 504 with adjustable heights without removal and/or replacement of distal end 504 of housing 202.

While FIGS. 1-6B describe device 104 with respect to device 104 configured to be implanted wholly within heart 104, the same structures and components described herein may be used to fix another implantable medical device within tissue of a patient. For example, an implantable medical device may include a fixation device similar to structure and/or function to helix and/or coil defined by first electrode 112 and anti-rotation features similar to any example ramps (e.g., ramp 212, ramp 504) as described above. In such examples, ramps 212 may prevent inhibit unintended rotation of a fixation helix and/or coil extending from a distal end of the fixation device.

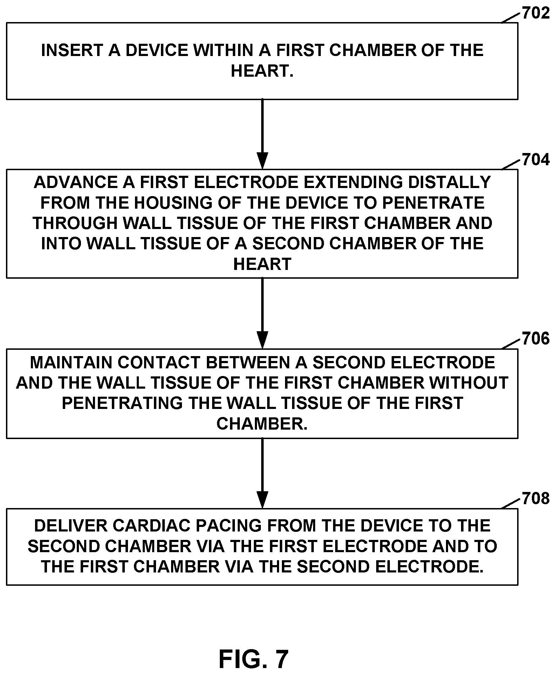

FIG. 7 is a flow diagram illustrating an example process for sensing a cardiac electrical signal and delivering cardiac pacing therapy to a heart of a patient via an device 104 of any of FIGS. 1-6B. The technique of FIG. 7 will be described with concurrent reference to device 104 as illustrated in FIGS. 1-2C, although a person having ordinary skill in the art will understand that the technique may be performed in reference to another implantable medical lead or other medical device.

A clinician may insert device 104 within a single first chamber of the heart 102 (702). The first chamber of heart 102 may be the right atrium, left atrium, the right ventricle, or the left ventricle. The clinician may insert device 104 into the first chamber via delivery tool connected to device 104 (e.g., to delivery tool interface member 208). The clinician may advance first electrode 112 extending distally from housing 202 of device 104 to penetrate through wall tissue of the first chamber and into wall tissue of a second chamber of heart 102 (704). In some examples, advancing first electrode 112 includes positioning a distal end of first electrode 112 (e.g., a first electrically active region 216) within a ventricular myocardium 108 of the patient. The clinician may advance first electrode 112 by rotating device 104 clockwise or counterclockwise within the first chamber, depending on how first electrode 112 is wound.

The clinician may cause device 104 to maintain contact between second electrode 114 and the wall tissue of the first chamber, without penetrating the wall tissue of the first chamber (706). The clinician may advance device 104 into the wall tissue until the wall tissue contacts ramp 212 and/or face 205 of housing 202. In some examples, when second electrode is disposed on ramp 212, ramp 212 may extend second electrode 114 distally from face 205 (e.g., from distal end 204) and along longitudinal axis 210 by distance 226. Ramp 212 may cause second electrode 114 to be placed relatively deeper within wall tissue than face 205 without penetrating the wall tissue, thereby allowing the wall tissue to at least partially envelop a distal surface and/or sides of second electrode 114. In some examples, ramp 212 causes second electrode 114 to maintain contact with the wall tissue of the first chamber (e.g., atrial endocardium 404 of the patient). Ramp 212 may place second electrode 114 in consistent contact with the wall tissue of the first chamber, thereby increasing the consistency of the sensing and/or pacing functionalities of second electrode 114.

While device 104 is implanted within the cardiac tissue, the one or more anti-rotation features defined resist rotation of device 104 due to movement of the cardiac tissue and blood flow. The one or more anti-rotation features may prevent inhibit movement of first electrode 112 away from wall tissue of the second chamber of heart 102 (e.g., ventricular myocardium 108 of the patient). The one or more anti-rotation features may also prevent inhibit dislodgement of device 104 from within wall tissue of the first chamber of heart 102. The one or more anti-rotation features may include ramp 212 maintaining contact with the wall tissue of the first chamber. Ramp 212 may increase compression of the wall tissue which causes device 104 to resist rotation due to movement of the wall tissue. The anti-rotation features may include one or more additional features (e.g., hooks, barbs, recesses, textured surfaces) disposed on one or more of first electrode 112, ramp 212, and/or face 205 of device 104.

The clinician may deliver cardiac pacing from device 104 to the second chamber via first electrode 112 and to the first chamber via second electrode 114 (708). Device 104 may deliver cardiac pacing to the first chamber and/or the second chamber via first electrode 112, second electrode 114, and/or one or more other electrodes of device 104 (e.g., electrode 218).

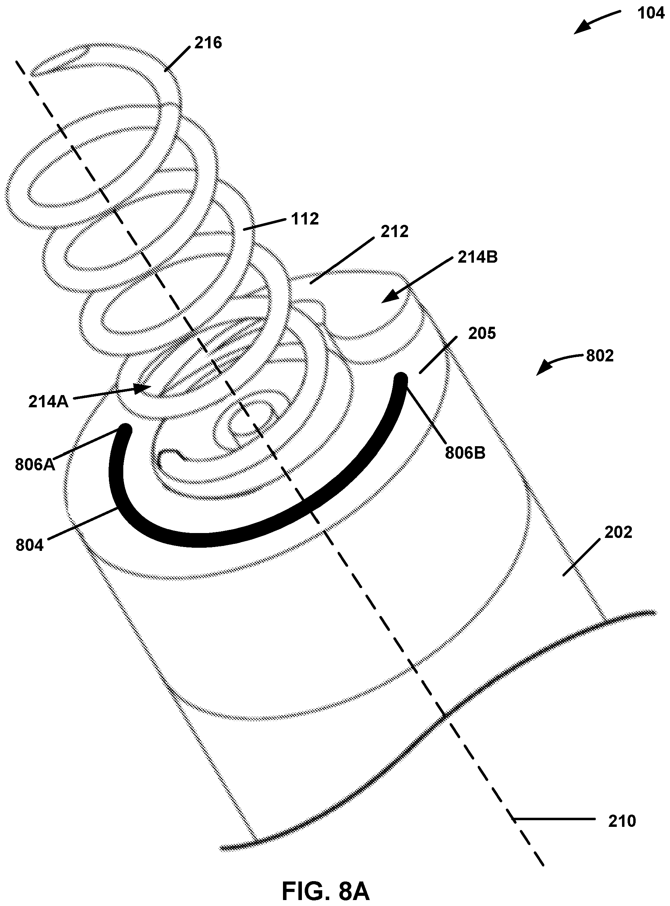

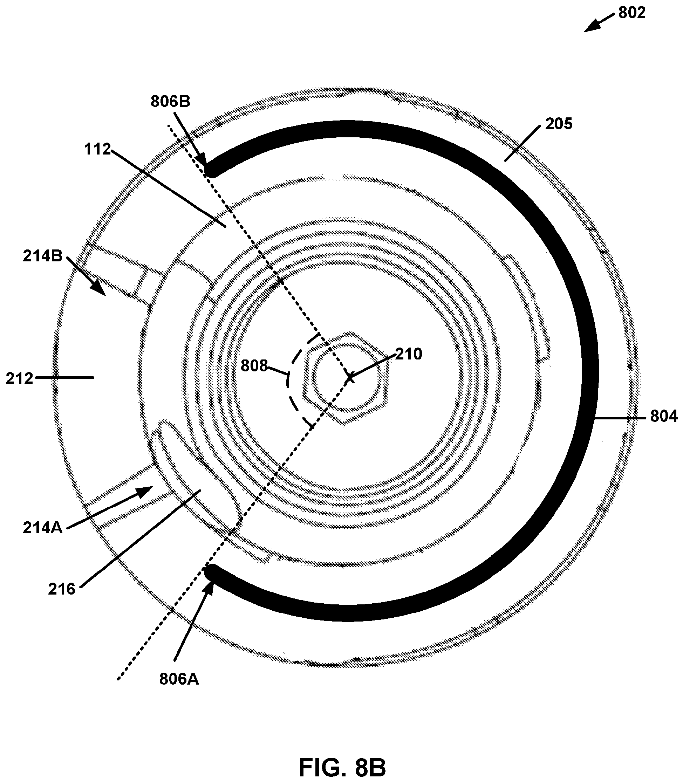

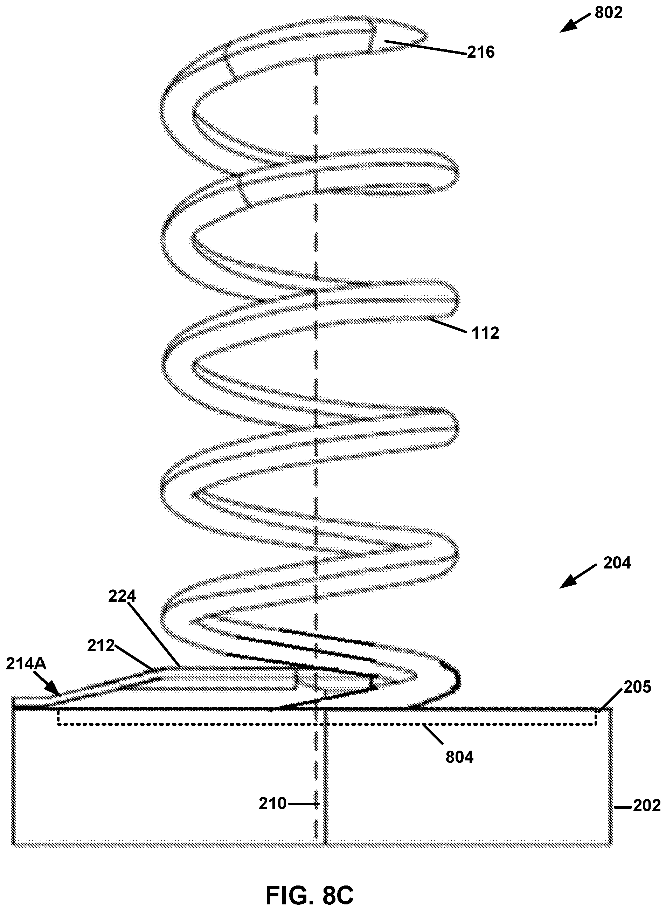

FIG. 8A is a perspective diagram illustrating another example distal end 802 of the example device 104 of FIG. 1. FIG. 8B is a perspective diagram illustrating a top-down view of example distal end 802 of example device 104 of FIG. 8A. FIG. 8C is a perspective diagram illustrating a side view of distal end 802 of the example device 104 of FIG. 8A. Distal end 802 may include first electrode 112, ramp 212, and second electrode 804 disposed on face 205 of distal end 802. Second electrode 804 may extend from a first end 806A to a second end 806B. In some examples, second electrode 804 is configured to extend at least partially around longitudinal axis 210 of device 104.

Second electrode 804 may include, but is not limited to, a button electrode or a wire electrode. Second electrode 802 may be formed from an electrically conductive, bio-compatible metallic alloy. For example, second electrode 804 may be formed from a Platinum Iridium alloy, a Titanium Nitride alloy, a Tantalum alloy, or the like. The cross-section of second electrode 804 may define a substantially round, rectangular, or flat ribbon shape. Second electrode 804 may define a uniform or varied cross-section along the length of second electrode 804. The cross-section and/or the variance in the cross-section of second electrode 804 may be select to control the electrically-conductive surface area of second electrode 804. For example, second electrode 804 with a round cross-section may define a smaller electrically-conductive surface area than another identical second electrode 804 with a rectangular cross-section.

Second electrode 804 may be disposed on face 205, e.g., such that a distal most surface of second electrode 804 extends distally away from face 205. In such examples, second electrode 804 may protrude from face 205 by a distance up to the thickness of second electrode 804 as measured along longitudinal axis 210. In some examples, second electrode 804 protrudes from face 205 by a distance of at least the thickness of second electrode 804. In such examples, second electrode 804 may define a gap between second electrode 804 and face 205. In some examples, second electrode 804 may be disposed on a distal surface of a protrusion (e.g., a ramp 212) extending from face 205. In some examples, e.g., as illustrated in FIG. 8C, second electrode 804 is disposed within a groove on face 205, e.g., such that the distalmost surface of second electrode 804 is flush with face 205. Second electrode 804 may define an uniform or varying width from first end 806A to second end 806B. In some examples, second electrode 804 includes one or more anti-fixation features (e.g., protrusions, texturing) disposed on the distalmost surface of second electrode 804, e.g., to inhibit unintended rotation of device 104. On distal end 802, ramp 212 may be positioned at a same or substantially similar location as illustrated with respect to distal end 204 as illustrated in FIGS. 2B-2C. Ramp 212 and second electrode 804 may be disposed on opposite and/or same quadrants and/or hemispheres of face 205 of distal end 802.

At least some portions of second electrode 804 (e.g., edges of second electrode 804) may be electrically insulated, e.g., to control an electrically conductive surface area of second electrode 804. The electrically conductive surface area of second electrode 804 may be substantially similar to the electrically conductive surface area of second electrical active region 217 of second electrode 114. In some examples, the electrically conductive surface area may be about 1.5 square-millimeters (mm²) to about 3.5 mm². For example, an second electrode 804 with an increased arc length may be thinner and/or may define a thinner electrically conductive surface than another second electrode 804, e.g., to maintain a same electrically conductive surface area.

As illustrated in FIG. 8B, second electrode 804 extends from first end 806A to second end 806B. First end 806A and second end 806B may be separated by an angle 808. Angle 808 may be up to 210 degrees (e.g., up to 165 degrees, 180 degrees, 201 degrees). In some examples, angle 808 may be greater than or equal to 20 degrees. As illustrated in FIGS. 8A and 8B, second electrode 804 may be separated from ramp 212, e.g., such that no portion of second electrode 804 is disposed on ramp 212 between first end 214A and second 214B. In some examples, one end of second electrode 804 (e.g., second end 806B), may be in contact with one end of ramp 212 (e.g., first end 214A).

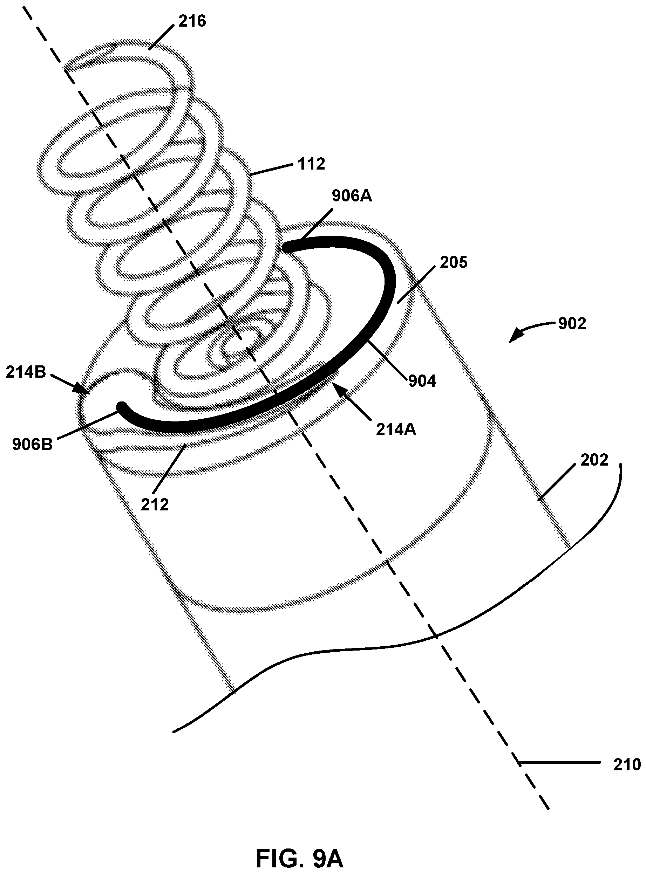

FIG. 9A is a perspective diagram illustrating another example distal end 902 of example device 104 of FIG. 1. Distal end 902 may include first electrode 112, ramp 212, and second electrode 904 disposed on face 205. Ramp 212 may extend from first end 214A and second end 214B, wherein second end 214B is more distal to face 205 than first end 214A. Ramp 212 may define a distalmost surface between first end 214A and second end 214B. Second electrode 904 extends from first end 906A to second end 906B. First end 906A and second end 906B may be collectively referred to herein as "ends 906." Second electrode 904 may extend at least partially around longitudinal axis 210. Second electrode 904 may extend at least partially along the distalmost surface of ramp 212.

In some examples, as illustrated in FIG. 9A, one end (e.g., second end 906B) of second electrode 904 is disposed on the distalmost surface of ramp 212 and an opposite end of second electrode 904 (e.g., first end 906A) is disposed on face 205. In some examples, second electrode 904 is entirely disposed on ramp 212 (e.g., ends 906 are disposed between first end 214A and second end 214B). second end 906B of second electrode 904 may be flush with second end 214B of ramp 212. In such examples, the sides of second electrode 904 at second end 906B may be placed in contact with cardiac tissue when device 104 is implanted within heart 102. In such examples, second electrode 904 may sense signals from and deliver cardiac pacing to cardiac tissue through the sides of second electrode 904 at second end 906B. Second electrode 904 may be disposed on face 205 in any of the manners previously described herein and may define same or similar materials and/or dimensions as second electrode 804. Electrode 804 or electrode 904 may be positioned on face 205 at any radial distance between an outer perimeter of the helix defined by first electrode 112 and an outer perimeter of housing 202. For example, an edge of electrode 804 or electrode 904 may be adjacent to an outer edge of housing 202 defining the outer perimeter of housing 202.

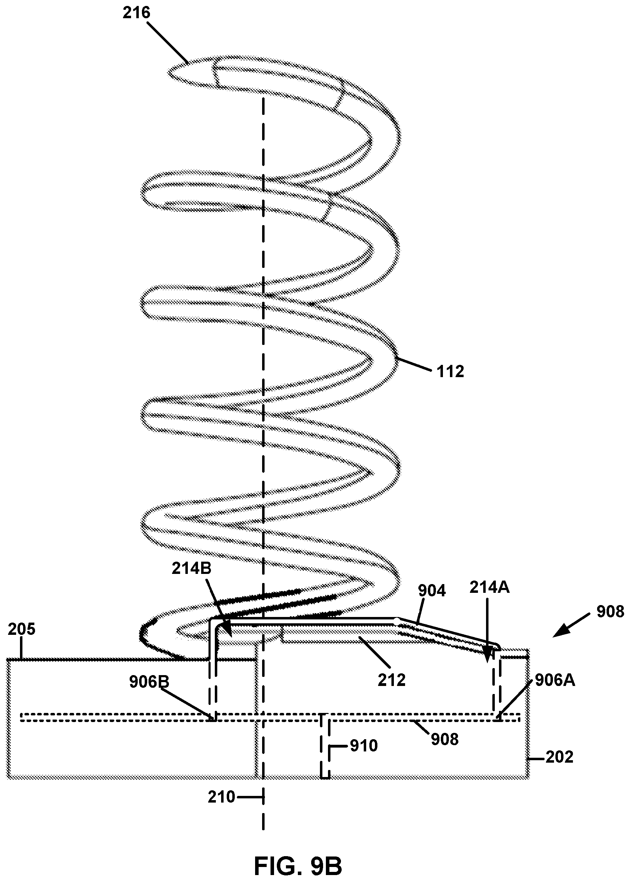

FIG. 9B is a perspective diagram illustrating a side view of another distal end of the example device of FIG. 1. Distal end 908 may be substantially similar to distal end 902 illustrated in FIG. 9A aside from the features described below.

As illustrated in FIG. 9B, second electrode 904 may extend from first end 906A and up and along the distalmost surface of ramp 212 from first end 214A to second end 214B. In such examples, second electrode 904 may define an electrically conductive surface along at least a portion of or the entire length of the distalmost surface of ramp 212. Device 104 may receive sensed signals from second electrode 904 and may determine based on the received signals whether the electrically conductive surface of second electrode 904, and therefore the distalmost surface of ramp 212, is in contact with cardiac tissue. Second electrode 904 may be disposed within ramp 212, e.g., such that the distal surface of second electrode 904 (e.g., the electrically conductive surface of second electrode 904) is flush with the distalmost surface of ramp 212. In some examples, second electrode 904 is disposed directly onto or on top of the distalmost surface of ramp 212.

In some examples, e.g., as illustrated in FIG. 9B, second end 906B of second electrode 904 extends over second end 214B of ramp 212 and back into housing 202. In such examples, both ends of second electrode 904 (e.g., first end 906A, second end 906B) may be retained within housing 202. A portion of second electrode 904 may extend from second end 214B of ramp 212 to face 205 of distal end 908 and may at least partially define the outer surface of ramp 212 at or around second end 214B. In such examples, the portion of second electrode 904 extending from second end 214B to face 205 may sense signals from or deliver cardiac pacing to cardiac tissue contacting one or more sides of ramp 212 when device 104 is implanted in heart 102.

Second electrode 904 may be electrically connected to one or more components disposed within housing 202 (e.g., to switch circuitry 302) via conducting element 908 and feedthrough assembly 910. While conducting element 908 and feedthrough assembly 910 are primarily described herein within respect to distal end 908 and second electrode 904 as illustrated in FIG. 9B, conducting element 908 and feedthrough assembly 910 may be used to couple any other electrodes described herein (e.g., first electrode 112, second electrode 114, second electrode 804) to component(s) within housing 202.

Conducting element 908 may be formed from an electrically conductive material and may be disposed within a portion of housing 202 defining the distal end (e.g., distal end 908) of device 104. Conducting element 908 may define a disc shape, an annular shape, or any other geometric shape sized to be fully retained within housing 202. Conducting element 908 may be, but is not limited to, a conducting plate, a conducting ring, a conducting wire, or the like. In some examples, as illustrated in FIG. 9B, conducting element 908 may extend orthogonal to longitudinal axis 210 of device 104. Conducting element 908 may be shaped and/or positioned within housing 202 to electrically isolate first electrode 112 from a second electrode (e.g., second electrode 114, 804, 904).

A feedthrough assembly 910 may extend from one or more components within housing 202 (e.g., from switch circuitry 302) to conducting element 908. Feedthrough assembly 910 electrically couples conducting element 908 to the one or more components and may transmit electrical signals between conducting element 908 and the one or more components. As illustrated in FIG. 9B, feedthrough assembly 910 may extend parallel to longitudinal axis 210 and may be radially and/or circumferentially offset from ends 906 of second electrode 904. For example, feedthrough assembly 910 may be disposed radially inward of ends 906 of second electrode 904, e.g., based on the positions and arrangement of components within housing 202.

Conducting element 908 may define one or more electrically conductive pathways coupling feedthrough assembly 910 to ends 906 of second electrode 904. Conducting element 908 may facilitate the formation of a complete electrical circuit between feedthrough assembly 910 and ends 906 of second electrode 904. Conducting element 908 may remove the need for the use of angled or offset feedthrough assemblies within device 104 and facilitate the use of feedthrough assembly 910 extending parallel to longitudinal axis 210. Conducting element 908 may also reduce the transference of torque and/or force to feedthrough assembly 910 during implantation and/or removal of device 104 and/or in response to movement of cardiac tissue during a cardiac cycle of heart 102. The ability to use feedthrough assembly 910 parallel to longitudinal axis 210 and the reduction in torque and/or force transference to feedthrough assembly 910 may reduce fatigue of feedthrough assembly 910 over time and reduce and/or inhibit a likelihood of failure of feedthrough assembly 910, e.g., due to fatigue, due to the application of excessive force or torque.

Second electrodes 804 and 904 illustrated in FIGS. 8A-9B may provide several technical advantages over other electrodes. In some examples, second electrodes 804 and 904 may further reduce unnecessary compression of cardiac tissue at target implantation region 106, which may improve capture thresholds (e.g., reduce capture thresholds) for the cardiac tissue at target implantation region 106, e.g., for atrial tissue at target implantation region 106). Second electrodes 804 and 904 may also define an increased footprint along face 205 compared to other electrodes, which may reduce dependency on orientation of device 104 to establish electrical contact between electrodes of device 104 and the tissue at target implantation region 106. The increased footprint may facilitate increased contact between second electrodes 804 and 904 and the tissue without increasing compression of the tissue Second electrodes 804 and 904 may be used in conjunction with one or more anti-rotation and/or fixation features for device 104, including, but is not limited to, fixation meshes, fixation recesses, fixation channels, protrusions, texturing, elongate darts, barbs, tines, or other tissue retention features.

It should be understood that various aspects disclosed herein may be combined in different combinations than the combinations specifically presented in the description and accompanying drawings. It should also be understood that, depending on the example, certain acts or events of any of the processes or methods described herein may be performed in a different sequence, may be added, merged, or left out altogether (e.g., all described acts or events may not be necessary to carry out the techniques). In addition, while certain aspects of this disclosure are described as being performed by a single module or unit for purposes of clarity, it should be understood that the techniques of this disclosure may be performed by a combination of units or modules associated with, for example, a medical device.

In one or more examples, the described techniques may be implemented in hardware, software, firmware, or any combination thereof. If implemented in software, the functions may be stored as one or more instructions or code on a computer-readable medium and executed by a hardware-based processing unit. Computer-readable media may include non-transitory computer-readable media, which corresponds to a tangible medium such as data storage media (e.g., RAM, ROM, EEPROM, flash memory, or any other medium that can be used to store desired program code in the form of instructions or data structures and that can be accessed by a computer).

In addition, it should be noted that system described herein may not be limited to treatment of a human patient. In alternative examples, the system may be implemented in non-human patients, e.g., primates, canines, equines, pigs, and felines. These other animals may undergo clinical or research therapies that may benefit from the subject matter of this disclosure.

Instructions may be executed by one or more processors, such as one or more digital signal processors (DSPs), general purpose microprocessors, application specific integrated circuits (ASICs), field programmable logic arrays (FPGAs), or other equivalent integrated or discrete logic circuitry. Accordingly, the term "processor" as used herein may refer to any of the foregoing structure or any other physical structure suitable for implementation of the described techniques. Also, the techniques could be fully implemented in one or more circuits or logic elements.

## Claims

1. A device (104) comprising:
an elongated housing (202) extending from a proximal end (206) to a distal end (204), the elongated housing being configured to be implanted wholly within a chamber of a heart (102);
a first electrode (112) extending distally from the distal end of the elongated housing, the first electrode comprising:
an elongated body defining a helix;
a ramp (212) extending distally from the distal end of the elongated housing; and
a second electrode (114) disposed on the ramp, wherein the ramp is configured to promote contact between the second electrode and wall tissue of the chamber without penetration of the wall tissue of the chamber by the second electrode, and wherein the ramp is configured to separate the second electrode from the distal end of the elongated housing by a fixed distance.

2. The device of claim 1, wherein the ramp defines a partial helix.

3. The device of any of claims 1 and 2, wherein the ramp extends from a first end (214A) to a second end (214B) around at least a portion of the perimeter of the elongated housing, and wherein the second end is more distal to the distal end of the elongated housing than the first end.

4. The device of any of claims 1-3, wherein the ramp extends at least partially around a longitudinal axis (210) of the elongated housing.

5. The device of any of claims 1-4, wherein the elongated body extends from the distal end of the elongated housing at a proximal end of the elongated body, wherein the proximal end of the elongated body and a radial center of the distal end of the elongated housing defines a first axis, wherein a center of the second electrode and the radial center of the distal end of the elongated housing defines a second axis, and wherein the first axis and the second axis are separated by a predetermined angle (222).

6. The device of claim 5, wherein the predetermined angle is less than or equal to 90 degrees.

7. The device of claim 5, wherein the predetermined angle is between 180 degrees and 360 degrees.

8. The device of any of claims 5-7, wherein a helix location at the predetermined angle is separated from the distal end by a threshold distance.

9. The device of any of claims 1-8, wherein the ramp comprises a first ramp, the device further comprising a second ramp extending distally from the distal end of the elongated housing.

10. The device of any of claims 1-9, wherein the second electrode is disposed on a distalmost surface of the ramp.

11. The device of any of claims 1-10, wherein the ramp defines a recess, and wherein the second electrode is disposed within the recess.

12. The device of claim 11, wherein a distalmost surface of the second electrode is flush with a distalmost surface of the ramp.

13. The device of any of claims 1-12, wherein the chamber of the heart comprises a first chamber of the heart, and wherein the helix is configured to penetrate into wall tissue of a second chamber of the heart that is separated from the first chamber of the heart.

14. The device of any of claims 1-13, wherein the ramp is configured to inhibit unintended rotation of the elongated body within the wall tissue of the second chamber.

15. The device of any of claims 1-14, wherein the distal end of the elongated housing further comprises one or more features configured to inhibit unintended rotation of the elongated body with the wall tissue of the second chamber.

## Patentansprüche

1. Vorrichtung (104), umfassend:
ein längliches Gehäuse (202), das sich von einem proximalen Ende (206) zu einem distalen Ende (204) erstreckt, wobei das längliche Gehäuse dazu ausgelegt ist, vollständig in eine Kammer eines Herzens (102) implantiert zu sein;
eine erste Elektrode (112), die sich entfernt von dem distalen Ende des länglichen Gehäuses erstreckt, wobei die erste Elektrode Folgendes umfasst:
einen länglichen Körper, der eine Spirale definiert;
eine Rampe (212), die sich entfernt von dem distalen Ende des länglichen Gehäuses erstreckt; und
eine zweite Elektrode (114), die auf der Rampe angeordnet ist, wobei die Rampe dazu ausgelegt ist, einen Kontakt zwischen der zweiten Elektrode und Wandgewebe der Kammer zu begünstigen, ohne dass die zweite Elektrode in das Wandgewebe der Kammer eindringt, und wobei die Rampe dazu ausgelegt ist, die zweite Elektrode von dem distalen Ende des länglichen Gehäuses um einen festen Abstand zu trennen.

2. Vorrichtung nach Anspruch 1, wobei die Rampe eine Teilspirale definiert.

3. Vorrichtung nach einem der Ansprüche 1 und 2, wobei sich die Rampe von einem ersten Ende (214A) zu einem zweiten Ende (214B) um zumindest einen Abschnitt des Umfangs des länglichen Gehäuses herum erstreckt, und wobei das zweite Ende weiter von dem distalen Ende des länglichen Gehäuses entfernt ist als das erste Ende.

4. Vorrichtung nach einem der Ansprüche 1-3, wobei sich die Rampe zumindest teilweise um eine Längsachse (210) des länglichen Gehäuses herum erstreckt.

5. Vorrichtung nach einem der Ansprüche 1-4, wobei sich der längliche Körper von dem distalen Ende des länglichen Gehäuses an einem proximalen Ende des länglichen Körpers erstreckt, wobei das proximale Ende des länglichen Körpers und ein radiales Zentrum des distalen Endes des länglichen Gehäuses eine erste Achse definieren, wobei ein Zentrum der zweiten Elektrode und das radiale Zentrum des distalen Endes des länglichen Gehäuses eine zweite Achse definieren, und wobei die erste Achse und die zweite Achse um einen vorbestimmten Winkel (222) getrennt sind.

6. Vorrichtung nach Anspruch 5, wobei der vorbestimmte Winkel kleiner als oder gleich 90 Grad ist.

7. Vorrichtung nach Anspruch 5, wobei der vorbestimmte Winkel zwischen 180 Grad und 360 Grad beträgt.

8. Vorrichtung nach einem der Ansprüche 5-7, wobei eine Spiralposition an dem vorbestimmten Winkel um einen Schwellenabstand von dem distalen Ende getrennt ist.

9. Vorrichtung nach einem der Ansprüche 1-8, wobei die Rampe eine erste Rampe umfasst, wobei die Vorrichtung ferner eine zweite Rampe umfasst, die sich entfernt von dem distalen Ende des länglichen Gehäuses erstreckt.

10. Vorrichtung nach einem der Ansprüche 1-9, wobei die zweite Elektrode auf einer am weitesten entfernten Oberfläche der Rampe angeordnet ist.

11. Vorrichtung nach einem der Ansprüche 1-10, wobei die Rampe eine Vertiefung definiert, und wobei die zweite Elektrode innerhalb der Vertiefung angeordnet ist.

12. Vorrichtung nach Anspruch 11, wobei eine am weitesten entfernte Oberfläche der zweiten Elektrode bündig mit einer am weitesten entfernten Oberfläche der Rampe ist.

13. Vorrichtung nach einem der Ansprüche 1-12, wobei die Herzkammer eine erste Herzkammer umfasst, und wobei die Spirale dazu ausgelegt ist, in Wandgewebe einer zweiten Herzkammer einzudringen, die von der ersten Herzkammer getrennt ist.

14. Vorrichtung nach einem der Ansprüche 1-13, wobei die Rampe dazu ausgelegt ist, eine unbeabsichtigte Drehung des länglichen Körpers innerhalb des Wandgewebes der zweiten Kammer zu verhindern.

15. Vorrichtung nach einem der Ansprüche 1-14, wobei das distale Ende des länglichen Gehäuses ferner ein oder mehrere Merkmale umfasst, die dazu ausgelegt sind, eine unbeabsichtigte Drehung des länglichen Körpers mit dem Wandgewebe der zweiten Kammer zu verhindern.

## Revendications

1. Dispositif (104) comprenant :
un boîtier allongé (202) s'étendant d'une extrémité proximale (206) à une extrémité distale (204), le boîtier allongé étant configuré pour être implanté entièrement dans une chambre d'un cœur (102) ;
une première électrode (112) s'étendant distalement à partir de l'extrémité distale du boîtier allongé, la première électrode comprenant :
un corps allongé définissant une hélice ;
une rampe (212) s'étendant distalement à partir de l'extrémité distale du boîtier allongé ; et
une seconde électrode (114) disposée sur la rampe, la rampe étant configurée pour favoriser le contact entre la seconde électrode et le tissu de paroi de la chambre sans pénétration du tissu de paroi de la chambre par la seconde électrode, et la rampe étant configurée pour séparer la seconde électrode de l'extrémité distale du boîtier allongé d'une distance fixe.

2. Dispositif selon la revendication 1, la rampe définissant une hélice partielle.

3. Dispositif selon l'une quelconque des revendications 1 et 2, la rampe s'étendant d'une première extrémité (214A) à une seconde extrémité (214B) autour d'au moins une partie du périmètre du boîtier allongé, et la seconde extrémité étant plus distale par rapport à l'extrémité distale du boîtier allongé que la première extrémité.

4. Dispositif selon l'une quelconque des revendications 1 à 3, la rampe s'étendant au moins partiellement autour d'un axe longitudinal (210) du boîtier allongé.

5. Dispositif selon l'une quelconque des revendications 1 à 4, le corps allongé s'étendant depuis l'extrémité distale du boîtier allongé à une extrémité proximale du corps allongé, l'extrémité proximale du corps allongé et un centre radial de l'extrémité distale du boîtier allongé définissant un premier axe, un centre de la seconde électrode et le centre radial de l'extrémité distale du boîtier allongé définissant un second axe, et le premier axe et le deuxième axe étant séparés d'un angle prédéterminé (222).

6. Dispositif selon la revendication 5, l'angle prédéterminé étant inférieur ou égal à 90 degrés.

7. Dispositif selon la revendication 5, l'angle prédéterminé étant compris entre 180 degrés et 360 degrés.

8. Dispositif selon l'une quelconque des revendications 5 à 7, un emplacement d'hélice à l'angle prédéterminé étant séparé de l'extrémité distale d'une distance seuil.

9. Dispositif selon l'une quelconque des revendications 1 à 8, la rampe comprenant une première rampe, le dispositif comprenant en outre une seconde rampe s'étendant distalement à partir de l'extrémité distale du boîtier allongé.

10. Dispositif selon l'une quelconque des revendications 1 à 9, la seconde électrode étant disposée sur une surface la plus distale de la rampe.

11. Dispositif selon l'une quelconque des revendications 1 à 10, la rampe définissant un évidement, et la seconde électrode étant disposée à l'intérieur de l'évidement.

12. Dispositif selon la revendication 11, une surface la plus distale de la seconde électrode affleurant une surface la plus distale de la rampe.

13. Dispositif selon l'une quelconque des revendications 1 à 12, la chambre du cœur comprenant une première chambre du cœur, et l'hélice étant configurée pour pénétrer dans le tissu de paroi d'une seconde chambre du cœur qui est séparée de la première chambre du cœur.

14. Dispositif selon l'une quelconque des revendications 1 à 13, la rampe étant configurée pour empêcher une rotation involontaire du corps allongé à l'intérieur du tissu de paroi de la seconde chambre.

15. Dispositif selon l'une quelconque des revendications 1 à 14, l'extrémité distale du boîtier allongé comprenant en outre une ou plusieurs caractéristiques configurées pour empêcher la rotation involontaire du corps allongé avec le tissu de paroi de la seconde chambre.
